# EUROPEAN PATENT APPLICATION

(11) **EP 4 553 502 A1**
(43) Date of publication of application: **14.05.2025**
(21) Application number: 23835882.4
(22) Date of filing: 07.07.2023
(51) Int. Cl.: G01N 33/574, C12Q 1/6869, C12N 9/22

(54) **METHOD FOR DISCOVERING CELL SURFACE ANTIGENS FOR NOVEL ANTIBODIES**

(30) Priority: 07.07.2022 KR 20220083514
(71) Applicant: The Asan Foundation, Seoul 05505 (KR); UNIVERSITY OF ULSAN FOUNDATION FOR INDUSTRY COOPERATION, Nam-gu Ulsan 44610 (KR)
(72) Inventor: CHANG, Su Hwan, Namyangju-si, Gyeonggi-do 12222 (KR); KIM, Yong Sub, Seoul 05505 (KR); CHOI, Eun A, Daegu 41403 (KR)
(74) Representative: AWA Sweden AB
(86) International application number: PCT/KR2023/009648
(87) International publication number: WO 2024/010416

(57) **Abstract**

The present disclosure relates to a method for discovering cell surface antigens for novel antibodies. According to a screening method of an aspect, cell surface antigens that bind to novel antibodies may be accurately screened. Moreover, the method is effective in efficiently screening cell surface antigens for novel antibodies by using cells that bind well to the novel antibodies.

## Description

### Technical Field

The present disclosure relates to a method for discovering cell surface antigens for novel antibodies.

### Background Art

Cell therapy using chimeric antigen receptors (CARs) is emerging as a promising cancer therapy method, and there is active research on personalized anticancer vaccines for patients that can increase effectiveness of immunotherapy by inducing the patient's immune response to be concentrated on cancer cell-specific neoantigens. In such anticancer therapy, screening effective antigens is the key technology.

In general, cDNA library screening methods have been used to identify neoantigens. These methods involve overexpressing cDNA libraries and MHC molecules in cell lines, followed by co-culturing with T cells for antigen identification that would induce activation of T cells. However, there are disadvantages of being labor-intensive, expensive, and difficult to identify all tumor antigens.

In addition, since immunoprecipitation-LC-MS/MS which is a commonly used method for discovering antigens also has low efficiency, there is currently no technology that can effectively discover cell surface antigens for novel antibodies.

Therefore, in antigen-based anticancer therapy, the current inefficient antigen discovery technology is considered as a technical obstacle, and technology that can effectively discover antigens is required.

In this regard, while conducting research on this basis, the inventors of the present disclosure constructed a guide RNA library for cell surface proteins and introduced it together with Cas9 into cancer cells, thereby completing the present disclosure by finding out possibility of effective discovery of cell surface antigens.

### Description

### Technical Problem

One aspect provides a method for screening a cell surface antigen, the method comprising: treating separated cells with a vector to which a guide RNA (gRNA) library for cell surface proteins of the separated cells is introduced to produce vector-treated cells; treating the vector-treated cells with a protein having binding ability to the separated cells to produce protein-treated cells; and obtaining, from the protein-treated cells, cells that have lost binding ability to the protein used in the treating.

### Technical Solution

One aspect provides a method for screening a cell surface antigen, the method comprising treating separated cells with a vector to which a guide RNA (gRNA) library for cell surface proteins of the separated cells is introduced to produce vector-treated cells; treating the vector-treated cells with a protein having binding ability to the separated cells to produce protein-treated cells; and obtaining, from the protein-treated cells, cells that have lost binding ability to the protein used in the treating.

The separated cells may be cancer cells.

The term "cancer" as used in the present specification refers to a physiological condition in animals that is typically characterized by abnormal or uncontrolled cell growth. Cancer may be, for example, associated with metastasis, interference with normally functioning surrounding cells, release of cytokines or other secretory products at abnormal levels, suppression or enhancement of inflammatory or immunological responses, neoplasia, premalignancy, malignancy, invasion of nearby or distant tissues or organs, such as lymph nodes, or the like. Cancer tissue may be separated from cancer. Obtaining cancer tissue from cancer may be done by a conventional anatomical method, for example, by cutting tissues present in cancer into several pieces with sterilized scissors. The cancer tissue thus obtained may be then washed with a serum-free medium or a phosphate buffered saline (PBS) containing antibiotics such as penicillin, streptomycin, or gentamicin, so as to remove contaminants including blood or the like present in the tissue. The cancer tissue separated as described above may be directly treated with an enzyme, or may be treated with an enzyme after the cancer tissue is further cut into smaller pieces by using sterilized scissors or the like.

In an embodiment, the cancer may be blood cancer or solid cancer, and the solid cancer may be at least one selected from the group consisting of lung cancer, skin cancer, stomach cancer, intestinal cancer, colon cancer, pancreatic cancer, liver cancer, thyroid cancer, uterine cancer, cervical cancer, ovarian cancer, testicular cancer, prostate cancer, breast cancer, and oral cancer, but is not limited thereto.

In an embodiment, the separated cells may include those including a Cas9 polypeptide. The Cas polypeptide may be one of protein components of a CRISPR/Cas system, and may be an activated endonuclease or a nick-forming enzyme. The Cas polypeptide may exhibit its activity by forming a complex with CRISPR RNA (crRNA) and trans-activating crRNA (tracrRNA). The separated cell may further include a Cas polynucleotide, which is a nucleic acid sequence encoding the Cas polypeptide.

The Cas polynucleotide may be a polynucleotide derived from a bacterium of the genus *Streptococcus* (e.g., *Streptococcus pyogenes*), the genus *Neisseria* (e.g., *Neisseria meningitidis*), the genus *Pasteurella* (e.g., *Pasteurella multocida*), the genus *Francisella* (e.g., *Francisella novicida*), or the genus *Campylobacter* (*e.g., Campylobacter jejuni*).

The Cas polypeptide may be a wild-type Cas polypeptide or a mutant Cas polypeptide. The mutant Cas polypeptide may be, for example, a polypeptide in which a catalytic aspartate residue is changed to another amino acid (e.g., alanine). The Cas polypeptide may be a recombinant protein.

The term "guide RNA (gRNA)" as used in the present specification refers to a polynucleotide that cuts, inserts, or links a target DNA within a cell through RNA editing. The gRNA may be single-chain gRNA (sgRNA). The gRNA may be crRNA specific to a target nucleic acid sequence. The gRNA may further include a tracrRNA that interacts with a Cas9 nuclease. The tracrRNA may include a polynucleotide that forms a loop structure. The gRNA may have a length of 10 to 30 nucleotides. The length of the gRNA may be, for example, 10, 11, 12, 13, 14, 15, 16, 17, 18, 19, 20, 21, 22, 23, 24, 25, 26, 27, 28, 29, or 30 nucleotides.

The gRNA may include RNA, DNA, PNA, or a combination thereof. The gRNA may be chemically modified.

The gRNA may be a component of gene scissors (e.g., a programmable nuclease). The gene scissors refer to any type of nucleases that can recognize and cut specific locations in the genome. The gene scissors may be, for example, a transcription activator-like effector nuclease (TALEN), a zinc-finger nuclease, a meganuclease, an RNA-guided engineered nuclease (RGEN), Cpf1, and an Ago homolog (e.g., a DNA-guided endonuclease). The RGEN refers to a nuclease that includes, as components, gRNA and a Cas protein that are specific to target DNA. The polynucleotide may be, for example, a component of the RGEN.

The gRNA may remove a nucleic acid sequence encoding a KRAS polypeptide from the genome of a cell by non-homologous end-joining (NHEJ).

The term "library" as used in the present specification refers to a pool or population including two or more types of homogeneous substances having different properties. In this regard, an oligonucleotide library may be a pool or population including two or more types oligonucleotides, such as gRNA, having different nucleotide sequences, and/or a pool or population including two types of oligonucleotides having different target sequences.

The gRNA library may be a pool or population of gRNAs targeting genes of cell surface proteins. The gRNA library may be a pool or population of gRNAs targeting genes of 2,000 to 6,000 types of cell surface proteins. The gRNA may include 1 to 10 gRNAs per gene of cell surface proteins.

The term "vector" as used in the present specification refers to a vehicle, such as a genetic construct, that can deliver the gRNA into a cell, and the vector may include nucleotide sequences encoding each gRNA. The vector may be a viral vector or a plasmid vector.

In an embodiment, the vector may be a viral vector. The viral vector may be a retroviral vector, an adenoviral vector, a lentiviral vector, a herpes viral vector, a varicella virus vector, a rhabdovirus vector, an alphavirus vector, a flavivirus vector, or an adeno-associated viral vector. The vector may be an expression vector. The vector may be a constitutive expression vector or an inducible expression vector. The vector may include a packaging signal, a rev-response element (RRV), a woodchunk post-transcriptional regulatory element (WPRE), a central polypurine tract (cPPT), a promoter, an antibiotic-resistant gene, an operator, a repressor, a T2A peptide, a reporter gene, or a combination thereof. The promoter may include an U6 polymerase III promoter, an elongation factor 1α promoter, an H1 promoter, a cytomegalovirus promoter, or a combination thereof. The antibiotic-resistant gene may include a puromycin-resistant gene, a blasticidin-resistant gene, or a combination thereof. The repressor may be a tetracycline operator. The reporter gene may include a nucleic acid sequence encoding an enhanced green fluorescent protein. When present within a cell of a subject, the vector may include essential regulatory elements that are operably linked to an insert, i.e. an insert designed for expression of an oligonucleotide.

A method for delivering the vector to a cell for producing a library may be accomplished by using various methods known in the art. For example, various methods known in the art, such as calcium phosphate-DNA co-precipitation, DEAE-dextran-mediated transfection, polybrene-mediated transfection, electroshock therapy, microinjection, liposome fusion, lipofectamine, protoplast fusion, and the like, may be used. In addition, when using a viral vector, virus particles may be used to deliver a target substance, i.e. the vector, into a cell by means of infection. Furthermore, the vector may be introduced into a cell by using a genetic bombardment or the like.

In an embodiment, in the vector-treated cells, one vector may be introduced per cell. By adjusting a multiplicity of infection (MOI) level to 0.2 to 0.4, for example, 0.3, one vector may be introduced per cell.

In an embodiment, the method may include removing cells into which the vector has not been introduced.

The term "protein having binding ability to a cell" as used in the present specification refers to a protein that recognizes specifically a surface protein of a cell or a protein that binds specifically to a surface protein of a cell. Therefore, the protein having binding ability to a cell may be a protein that binds specifically to the cell surface protein.

In an embodiment, the protein that binds specifically to the cell surface protein may be any one selected from the group consisting of an antibody, an affibody, and a diabody.

The term "antibody" as used in the present specification refers to any antigen-binding molecule or molecular complex including at least one complementarity determining region (CDR) that binds specifically to or interacts with a particular antigen. The antibody may include not only immunoglobulin molecules including four polypeptide chains consisting of two heavy (H) chains and two light (L) chains that are interconnected by disulfide bonds, but also multimers of the immunoglobulin molecules (e.g., IgM). In addition, the antibody may include an immunoglobulin molecule consisting of four polypeptide chains consisting of two H chains and two L chains that are interconnected by disulfide bonds. Each H chain may include a heavy chain variable region (abbreviated herein as HCVR or VH) and a heavy chain constant region. The heavy chain constant region may include three domains, CH1, CH2 and CH3. Each L chain may include a light chain variable region (abbreviated herein as LCVR or VL) and a light chain constant region. The light chain constant region may include one domain (CL1). The VH and VL regions may be further subdivided into hypervariable regions, called complementarity determining regions (CDRs) that are interspersed with more conserved regions called framework regions (FRs). The VH and VL regions may each consist of three CDRs and four FRs, which are arranged from amino-terminus to carboxy-terminus in the following order: FR1, CDR1, FR2, CDR2, FR3, CDR3, FR4.

The antibody may also include an antigen-binding fragment of a whole antibody molecule. The terms "antigen-binding portion" of an antibody, "antigen-binding fragment" of an antibody, and the like may include any naturally occurring, enzymatically obtainable, synthetic, or genetically engineered polypeptide or glycoprotein that binds specifically to an antigen to form a composite. The antigen-binding fragment of an antibody may be, for example, derived from a whole antibody molecule by using any suitable standard technique, such as, proteolytic hydrolysis digestion, or recombinant genetic engineering techniques involving manipulation and expression of DNA encoding antibody variable and selective constant domains. Such DNA may be known in the art and/or readily available from, for example, commercially available DNA libraries (including phage-antibody libraries), or may be synthesized. The DNA may be, for example, sequenced and manipulated chemically or by using molecular biological techniques, to arrange one or more variable domains and/or constant domains into a suitable configuration, to introduce codons, to generate cysteine residues, to modify, add, or delete amino acids, and the like.

The term "affibody" as used in the present specification may refer to an antibody mimic capable of binding to a specific target protein (e.g., a receptor). Typically, the affibody molecule consists of 20 to 150 amino acid residues, and may consist of 2 to 10 alpha helices.

In an embodiment, the cell surface protein providing a binding site for the protein having binding ability to the separated cell may be a binding site to an Fc region of an antibody or antibody analog.

In an embodiment, the cell surface protein and the protein having binding ability to the separated cell may be linked by a non-covalent bond.

The term "cell surface protein" as used in the present specification may refer to a protein present on the surface of a cell. In an embodiment, the cell surface protein may be an antigen binding to the treated protein. Accordingly, an antigen that binds well to a novel antibody may be discovered through the screening method.

In an embodiment, the obtaining of the cells that have lost the binding ability to the treated protein may include: treating the protein-treated cells with a bead with a surface that binds to the treated protein; and obtaining cells that do not bind to the bead. The bead may have a surface modified to enable binding to the treated protein.

In an embodiment, the method may include: analyzing the gRNA contained in the cells that have lost binding ability to the treated protein; and identifying a gene targeted by the analyzed gRNA.

In an embodiment, the method may include: preparing a control cell in which the gene targeted by the analyzed gRNA is knocked down or knocked out; and treating the control cell with an antibody to measure whether an antigen-antibody reaction occurs. The control cell in which the gene targeted by the gRNA is knocked down may be prepared by introducing siRNA of a target gene.

In an embodiment, the antigen-antibody reaction may be measured by using any one selected from the group consisting of enzyme-linked immunosorbent assay, radioimmunoassay, sandwich assay, western blotting, immunoprecipitation, immunohistochemical staining, fluorescent immunoassay, enzyme-substrate chromogenic assay, and antigen-antibody agglutination.

In an embodiment, the cell surface proteins may include a tumor-associated antigen (TAA).

The term "tumor-associated antigen (TAA)" as used in the present specification may refer to any antigen including but not limited to proteins associated with cancer. Such an antigen may be expressed on malignant cells or in the tumor microenvironment, such as tumor-associated blood vessels, extracellular matrix, mesenchymal stroma, or immune infiltrates.

The TAA may be, for example, AFP, ALK, BAGE protein, BIRC5 (survivin), BIRC7, β-catenin, brc-abl, BRCA1, BORIS, CA9, carbonic anhydrase IX, caspase-8, CALR, CCR5, CD19, CD20(MS4A1), CD22, CD40, CD70, CDK4, CEA, cyclin-B1, CYP1B1, EGFR, EGFRvIII, ErbB2/Her2, ErbB3, ErbB4, ETV6-AML, EpCAM, EphA2, Fra-1, FOLR1, GAGE protein(for example, GAGE-1, -2), GD2, GD3, GloboH, glypican-3, GM3, gp100, Her2, HLA/B-raf, HLA/k-ras, HLA/MAGE-A3, hTERT, IL-10, LMP2, MAGE proteins (e.g., MAGE-1, -2, -3, -4, -6, and -12), MART-1, mesothelin, ML-IAP, Muc1, Muc2, Muc3, Muc4, Muc5, Muc16(CA-125), MUM1, NA17, NY-BR1, NY-BR62, NY-BR85, NY-ESO1, p15, p53, PAP, PAX3, PAX5, PCTA-1, PLAC1, PRLR, PRAME, PSMA(FOLH1), RAGE protein, Ras, RGS5, Rho, SART-1, SART-3, STEAP1, STEAP2, TAG-72, TGF-β, TMPRSS2, a thompson-nouvelle antigen(Tn), TRP-1, TRP-2, tyrosinase, or uroplakin-3.

### Advantageous Effects

By a screening method according to one aspect, a cell surface antigen binding to a novel antibody may be accurately screened, and through a cell that binds well to the novel antibody, a cell surface antigen for the antibody may be efficiently screened. Accordingly, the discovery of novel antibodies present in the serum of a patient may lead to discovery of novel major antigens, and the discovery of novel antigens may become a new therapeutic strategy to overcome resistance in anticancer therapy, resulting in important significance in the fields of anticancer therapy and immunotherapy and contributing to development of personalized therapy strategies for patients.

### Description of Drawings

FIG. 1 is an image for determining expression of Cas9 in breast cancer cells, wherein MDA-MB-468 is a breast cancer cell that is not transduced with Cas9, and MDA-MB-468-cas9 is a breast cancer cell that is transduced with Cas9.

FIG. 2 is a graph showing the results of performing MACS for Cas9/guide RNA library cells by using cetuximab as an antibody, confirming guide RNAs that are highly expressed in cells not labeled with cetuximab over cells labeled with cetuximab.

FIG. 3 is a graph showing the results of performing MACS by using a CD44 antibody, confirming guide RNAs, which are highly expressed in cells not labeled with the CD44 antibody, in different cell lines, wherein

FIG. 3A is a graph confirming guide RNAs, which are highly expressed in cells not labeled with the CD44 antibody, in a HeLa cell line, and FIG. 3B is a graph confirming guide RNAs, which are highly expressed in cells not labeled with the CD44 antibody, in an A549 cell line.

FIG. 4 is a graph showing the results of performing MACS for Cas9/guide RNA library cells by using, as antibodies, S4-2 and S3-5 anticancer antibodies discovered from a patient-derived antibody library, confirming guide RNAs that are highly expressed in cells not labeled with the S4-2 or S3-5 anticancer antibody over cells labeled with the S4-2 or S3-5 anticancer antibody, wherein

FIG. 4A is a graph confirming guide RNAs highly expressed in cells not labeled with the S4-2 anticancer antibody discovered from a patient-derived antibody library, and FIG. 4B is a graph confirming guide RNAs highly expressed in cells not labeled with the S3-5 anticancer antibody discovered from a patient-derived antibody library.

FIG. 5 is a graph showing the results of performing fluorescence-activated cell sorting (FACS) after treating an MDA-MB-468 cell line with ICAM1-specific siRNAs.

FIG. 6 is an image obtained by performing immunoprecipitation-western blotting on an HS578T breast cancer cell line expressing ICAM1.

FIG. 7 is an image obtained by performing immunoprecipitation-western blotting to determine whether ICAM1 directly binds to S4-2 and S3-5 anticancer antibodies.

FIG. 8 is a schematic diagram explaining a method for screening cell surface antigens.

### Mode for Invention

Hereinafter, the present disclosure will be described in more detail with reference to Examples below. However, these Examples are for illustrative purposes only, and the scope of the present disclosure is not intended to be limited by these Examples.

### Example 1. Construction of cells stably expressing Cas9

To construct cells stably expressing Cas9, 1 day before transfection, HEK293T cells were seeded at 70 % confluency. The HEK293T cells were co-transfected with pMD2.G (1.5 µg), psPAX2 (1.5 µg), and lentiCas9-Blast (4.5 µg) plasmids (e.g., packaging plasmids) by using Lipofectamine 3000 for packaging. 6 hours after the transfection, the medium was replaced with a DMEM medium supplemented with 10 % FBS and 1 % penicillin/streptomycin (P/S). Afterwards, the culture supernatant containing virus particles was collected every 24 hours and centrifuged at 1,200 rpm for 5 minutes to remove any remaining HEK293T cells. The collected supernatant containing virus was filtered through a 0.45 µm-filter.

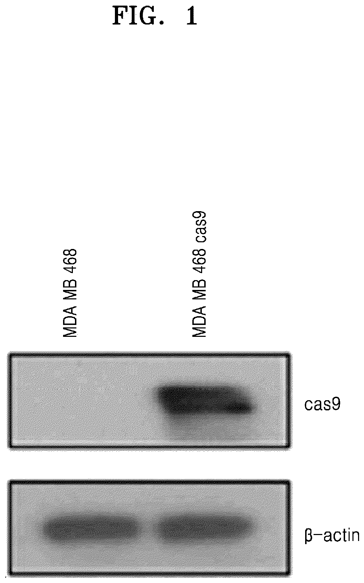

To produce breast cancer cells (MDA-MB-468) stably expressing Cas9, the medium containing virus was supplemented with polybrene (10 µg/ml) twice repeatedly. Following 24 hours of incubation, breast cancer cells (MDA-MB-468-cas9) stably expressing Cas9 were constructed with 6 to 8 µg/ml of blasticidine S hydrochloride (Sigma). Then, to determine whether Cas9 was well expressed in the constructed breast cancer cells, western blotting was performed, and the results are shown in FIG. 1.

FIG. 1 is an image for determining expression of Cas9 in breast cancer cells, wherein MDA-MB-468 is a breast cancer cell that is not transduced with Cas9, and MDA-MB-468-cas9 is a breast cancer cell that is transduced with Cas9.

As shown in FIG. 1, it was confirmed that the MDA-MB-468-cas9 transduced with Cas9 stably expressed Cas9.

### Example 2. Construction of guide RNA (gRNA) library for cell surface proteins

Regarding about 5,000 cell surface proteins, five gRNAs targeting each gene were designed and synthesized, and then cloned into a lentiviral vector to construct a gRNA library for cell surface proteins.

More specifically, by analyzing genes with well-verified genetic information among proteins known to exist on the surface of a cell, a total of 2,692 cell surface proteins were selected and shown in Table 1 (see Proc Natl Acad Sci USA, 2018 Nov 13;115(46): E10988-E10997 and HGNC database). Regarding about 5,000 cell surface proteins, five gRNAs targeting each gene were designed and synthesized, and then cloned into a lentiviral vector to construct a gRNA library for cell surface proteins.

More specifically, by analyzing genes with well-validated genetic information among proteins known to exist on the surface of a cell, a total of 2,692 cell surface proteins were selected and shown in Table 1 (see Proc Natl Acad Sci USA, 2018 Nov 13;115(46): E10988-E10997 and HGNC database).

**[Table 1]**

| Serial number | Protein | Serial number | Protein | Serial number | Protein | Serial number | Protein | Serial number | Protein |
|---|---|---|---|---|---|---|---|---|---|
| 1 | ABCA1 | 540 | DPEP3 | 1079 | KCNJ1 2 | 1618 | OR5J2 | 2157 | SLC4A 1 |
| 2 | ABCA2 | 541 | DPP4 | 1080 | KCNK5 | 1619 | OR5K1 | 2158 | SLC4A 4 |
| 3 | ABCA3 | 542 | DPP6 | 1081 | KCNK1 8 | 1620 | OR5K2 | 2159 | SLC4A 5 |
| 4 | ABCA4 | 543 | DPP10 | 1082 | KCNM B1 | 1621 | OR5K3 | 2160 | SLC4A 7 |
| 5 | ABCA5 | 544 | DRD1 | 1083 | KCNM B2 | 1622 | OR5K4 | 2161 | SLC4A 8 |
| 6 | ABCA6 | 545 | DRD2 | 1084 | KCNM B3 | 1623 | OR5L1 | 2162 | SLC4A 10 |
| 7 | ABCA7 | 546 | DRD3 | 1085 | KCNM B4 | 1624 | OR5L2 | 2163 | SLC5A 1 |
| 8 | ABCA8 | 547 | DRD4 | 1086 | KCNS1 | 1625 | OR5M 1 | 2164 | SLC5A 2 |
| 9 | ABCA9 | 548 | DRD5 | 1087 | KCNV2 | 1626 | OR5M 3 | 2165 | SLC5A 3 |
| 10 | ABCA1 2 | 549 | DSC1 | 1088 | KDR | 1627 | OR5M 8 | 2166 | SLC5A 4 |
| 11 | ABCA1 3 | 550 | DSC2 | 1089 | KEL | 1628 | OR5M 9 | 2167 | SLC5A 5 |
| 12 | ABCB1 | 551 | DSC3 | 1090 | KIAA03 19 | 1629 | OR5M 10 | 2168 | SLC5A 6 |
| 13 | ABCB4 | 552 | DSCA M | 1091 | KIAA13 24 | 1630 | OR5M 11 | 2169 | SLC5A 7 |
| 14 | ABCB5 | 553 | DSCA ML1 | 1092 | KIAA13 24L | 1631 | OR5P2 | 2170 | SLC5A 8 |
| 15 | ABCB9 | 554 | DSG1 | 1093 | KIAA15 49L | 1632 | OR5P3 | 2171 | SLC5A 9 |
| 16 | ABCB1 1 | 555 | DSG2 | 1094 | KIR2DL1 | 1633 | OR5R1 | 2172 | SLC5A 10 |
| 17 | ABCC1 | 556 | DSG3 | 1095 | KIR2D L3 | 1634 | OR5T1 | 2173 | SLC5A 11 |
| 18 | ABCC2 | 557 | DSG4 | 1096 | KIR2D L4 | 1635 | OR5T2 | 2174 | SLC5A 12 |
| 19 | ABCC3 | 558 | DUOX 1 | 1097 | KIR2D S4 | 1636 | OR5T3 | 2175 | SLC6A 1 |
| 20 | ABCC4 | 559 | DUOX 2 | 1098 | KIR3D L1 | 1637 | OR5V1 | 2176 | SLC6A 2 |
| 21 | ABCC5 | 560 | DUOX A1 | 1099 | KIR3D L2 | 1638 | OR5W 2 | 2177 | SLC6A 3 |
| 22 | ABCC9 | 561 | DYNA P | 1100 | KIR3D L3 | 1639 | OR6A2 | 2178 | SLC6A 4 |
| 23 | ABCC1 0 | 562 | EBP | 1101 | KIRRE L1 | 1640 | OR6B1 | 2179 | SLC6A 5 |
| 24 | ABCC1 1 | 563 | ECE1 | 1102 | KIRRE L2 | 1641 | OR6B2 | 2180 | SLC6A 6 |
| 25 | ABCC1 2 | 564 | ECSC R | 1103 | KIRRE L3 | 1642 | OR6B3 | 2181 | SLC6A 7 |
| 26 | ABCG2 | 565 | EDA | 1104 | KISS1 R | 1643 | OR6C1 | 2182 | SLC6A 8 |
| 27 | ABCG4 | 566 | EDA2R | 1105 | KIT | 1644 | OR6C2 | 2183 | SLC6A 9 |
| 28 | ABCG5 | 567 | EDAR | 1106 | KITLG | 1645 | OR6C3 | 2184 | SLC6A 11 |
| 29 | ACE | 568 | EDNR A | 1107 | KL | 1646 | OR6C4 | 2185 | SLC6A 12 |
| 30 | ACE2 | 569 | EDNR B | 1108 | KLRB1 | 1647 | OR6C6 | 2186 | SLC6A 13 |
| 31 | ACHE | 570 | EFNA1 | 1109 | KLRC1 | 1648 | OR6C6 5 | 2187 | SLC6A 14 |
| 32 | ACKR1 | 571 | EFNA2 | 1110 | KLRC2 | 1649 | OR6C6 8 | 2188 | SLC6A 15 |
| 33 | ACKR2 | 572 | EFNA3 | 1111 | KLRC3 | 1650 | OR6C7 0 | 2189 | SLC6A 16 |
| 34 | ACKR3 | 573 | EFNA4 | 1112 | KLRF1 | 1651 | OR6C7 4 | 2190 | SLC6A 17 |
| 35 | ACKR4 | 574 | EFNA5 | 1113 | KLRF2 | 1652 | OR6C7 5 | 2191 | SLC6A 18 |
| 36 | ACP2 | 575 | EFNB1 | 1114 | KLRG1 | 1653 | OR6C7 6 | 2192 | SLC6A 19 |
| 37 | ACP4 | 576 | EFNB2 | 1115 | KLRK1 | 1654 | OR6F1 | 2193 | SLC6A 20 |
| 38 | ACVR1 | 577 | EFNB3 | 1116 | KREM EN1 | 1655 | OR6J1 | 2194 | SLC7A 1 |
| 39 | ACVR1 B | 578 | EGF | 1117 | KREM EN2 | 1656 | OR6K2 | 2195 | SLC7A 2 |
| 40 | ACVR1 C | 579 | EGFR | 1118 | L1CAM | 1657 | OR6K3 | 2196 | SLC7A 3 |
| 41 | ACVR2 A | 580 | ELFN1 | 1119 | LAG3 | 1658 | OR6K6 | 2197 | SLC7A 4 |
| 42 | ACVR2 B | 581 | ELFN2 | 1120 | LAIR1 | 1659 | OR6M 1 | 2198 | SLC7A 5 |
| 43 | ACVRL 1 | 582 | EMB | 1121 | LAMP1 | 1660 | OR6N1 | 2199 | SLC7A 6 |
| 44 | ADAM 2 | 583 | EMCN | 1122 | LAMP2 | 1661 | OR6N2 | 2200 | SLC7A 9 |
| 45 | ADAM 7 | 584 | EMP1 | 1123 | LAMP3 | 1662 | OR6P1 | 2201 | SLC7A 10 |
| 46 | ADAM 8 | 585 | EMP2 | 1124 | LAMP5 | 1663 | OR6Q1 | 2202 | SLC7A 14 |
| 47 | ADAM 9 | 586 | EMP3 | 1125 | LAYN | 1664 | OR6S1 | 2203 | SLC8A 1 |
| 48 | ADAM 10 | 587 | ENG | 1126 | LCT | 1665 | OR6T1 | 2204 | SLC8A 2 |
| 49 | ADAM 11 | 588 | ENPEP | 1127 | LDLR | 1666 | OR6V1 | 2205 | SLC8A 3 |
| 50 | ADAM 12 | 589 | ENPP1 | 1128 | LDLRA D3 | 1667 | OR6X1 | 2206 | SLC8B 1 |
| 51 | ADAM 15 | 590 | ENPP4 | 1129 | LDLRA D4 | 1668 | OR6Y1 | 2207 | SLC9A 1 |
| 52 | ADAM 17 | 591 | ENPP5 | 1130 | LEPR | 1669 | OR7A5 | 2208 | SLC9A 2 |
| 53 | ADAM 18 | 592 | ENPP6 | 1131 | LGR4 | 1670 | OR7A1 0 | 2209 | SLC9A 3 |
| 54 | ADAM 19 | 593 | ENTPD 1 | 1132 | LGR5 | 1671 | OR7A1 7 | 2210 | SLC9A 6 |
| 55 | ADAM 20 | 594 | ENTPD 3 | 1133 | LGR6 | 1672 | OR7C1 | 2211 | SLC9A 7 |
| 56 | ADAM 21 | 595 | EPCA M | 1134 | LHCG R | 1673 | OR7C2 | 2212 | SLC10 A1 |
| 57 | ADAM 22 | 596 | EPGN | 1135 | LHFPL 1 | 1674 | OR7D2 | 2213 | SLC10 A2 |
| 58 | ADAM 23 | 597 | EPHA1 | 1136 | LHFPL 4 | 1675 | OR7D4 | 2214 | SLC10 A3 |
| 59 | ADAM 28 | 598 | EPHA2 | 1137 | LHFPL 5 | 1676 | OR7E2 4 | 2215 | SLC10 A4 |
| 60 | ADAM 20 | 599 | EPHA3 | 1138 | LIFR | 1677 | OR7G1 | 2216 | SLC10 A5 |
| 61 | ADAM 30 | 600 | EPHA4 | 1139 | LILRA1 | 1678 | OR7G2 | 2217 | SLC10 A6 |
| 62 | ADAM 32 | 601 | EPHA5 | 1140 | LILRA2 | 1679 | OR7G3 | 2218 | SLC11 A1 |
| 63 | ADAM 33 | 602 | EPHA6 | 1141 | LILRA4 | 1680 | OR8A1 | 2219 | SLC11 A2 |
| 64 | ADCY2 | 603 | EPHA7 | 1142 | LILRA5 | 1681 | OR8B2 | 2220 | SLC12 A1 |
| 65 | ADCY3 | 604 | EPHA8 | 1143 | LILRA6 | 1682 | OR8B3 | 2221 | SLC12 A2 |
| 66 | ADCY5 | 605 | EPHA1 0 | 1144 | LILRB1 | 1683 | OR8B4 | 2222 | SLC12 A3 |
| 67 | ADCY6 | 606 | EPHB1 | 1145 | LILRB2 | 1684 | OR8B8 | 2223 | SLC12 A4 |
| 68 | ADCY7 | 607 | EPHB2 | 1146 | LILRB3 | 1685 | OR8B1 2 | 2224 | SLC12 A5 |
| 69 | ADCY9 | 608 | EPHB3 | 1147 | LILRB5 | 1686 | OR8D1 | 2225 | SLC12 A6 |
| 70 | ADCYA P1R1 | 609 | EPHB4 | 1148 | LIM2 | 1687 | OR8D2 | 2226 | SLC12 A7 |
| 71 | ADGRA1 | 610 | EPHB6 | 1149 | LINGO1 | 1688 | OR8D4 | 2227 | SLC12 A8 |
| 72 | ADGR A2 | 611 | EPOR | 1150 | LINGO 2 | 1689 | OR8G1 | 2228 | SLC12 A9 |
| 73 | ADGR A3 | 612 | EQTN | 1151 | LINGO 3 | 1690 | OR8G5 | 2229 | SLC13 A1 |
| 74 | ADGR B1 | 613 | ERBB2 | 1152 | LINGO 4 | 1691 | OR8H1 | 2230 | SLC13 A2 |
| 75 | ADGR B2 | 614 | ERBB3 | 1153 | LMAN2 | 1692 | OR8H2 | 2231 | SLC13 A3 |
| 76 | ADGR B3 | 615 | ERBB4 | 1154 | LMAN2 L | 1693 | OR8H3 | 2232 | SLC13 A4 |
| 77 | ADGR D1 | 616 | EREG | 1155 | LMBR1 | 1694 | OR8I2 | 2233 | SLC14 A1 |
| 78 | ADGR D2 | 617 | ERMA P | 1156 | LMBR1 L | 1695 | OR8J1 | 2234 | SLC14 A2 |
| 79 | ADGR E1 | 618 | ERMP 1 | 1157 | LMBR D1 | 1696 | OR8J2 | 2235 | SLC15 A1 |
| 80 | ADGR E2 | 619 | ERVFR D-1 | 1158 | LMBR D2 | 1697 | OR8J3 | 2236 | SLC15 A2 |
| 81 | ADGR E3 | 620 | ERVM ER34-1 | 1159 | LNPEP | 1698 | OR8K1 | 2237 | SLC15 A3 |
| 82 | ADGR E5 | 621 | ERVV-1 | 1160 | LPAR1 | 1699 | OR8K3 | 2238 | SLC15 A4 |
| 83 | ADGR F1 | 622 | ERVV-2 | 1161 | LPAR2 | 1700 | OR8K5 | 2239 | SLC15 A5 |
| 84 | ADGR F2 | 623 | ERVW-1 | 1162 | LPAR3 | 1701 | OR8S1 | 2240 | SLC16 A1 |
| 85 | ADGR F3 | 624 | ESAM | 1163 | LPAR4 | 1702 | OR8U1 | 2241 | SLC16 A4 |
| 86 | ADGR F4 | 625 | ESYT3 | 1164 | LPAR5 | 1703 | OR9A2 | 2242 | SLC16 A5 |
| 87 | ADGR F5 | 626 | EVA1C | 1165 | LPAR6 | 1704 | OR9A4 | 2243 | SLC16 A6 |
| 88 | ADGR G1 | 627 | EVC2 | 1166 | LPL | 1705 | OR9G1 | 2244 | SLC16 A7 |
| 89 | ADGR G2 | 628 | EVI2A | 1167 | LRFN1 | 1706 | OR9G4 | 2245 | SLC16 A8 |
| 90 | ADGR G3 | 629 | EVI2B | 1168 | LRFN2 | 1707 | OR9I1 | 2246 | SLC16 A12 |
| 91 | ADGR G4 | 630 | F2R | 1169 | LRFN3 | 1708 | OR9K2 | 2247 | SLC17 A1 |
| 92 | ADGR G5 | 631 | F2RL1 | 1170 | LRFN4 | 1709 | OR9Q1 | 2248 | SLC17 A5 |
| 93 | ADGR G6 | 632 | F2RL2 | 1171 | LRFN5 | 1710 | OR9Q2 | 2249 | SLC17 A6 |
| 94 | ADGR G7 | 633 | F2RL3 | 1172 | LRIG1 | 1711 | OR10A 2 | 2250 | SLC17 A7 |
| 95 | ADGR L1 | 634 | F3 | 1173 | LRIG2 | 1712 | OR10A 3 | 2251 | SLC17 A8 |
| 96 | ADGR L2 | 635 | F11R | 1174 | LRIG3 | 1713 | OR10A 4 | 2252 | SLC17 A9 |
| 97 | ADGR L3 | 636 | FAIM2 | 1175 | LRIT1 | 1714 | OR10A 5 | 2253 | SLC18 A1 |
| 98 | ADGR L4 | 637 | FAM17 1A1 | 1176 | LRIT2 | 1715 | OR10A 6 | 2254 | SLC18 A2 |
| 99 | ADGR V1 | 638 | FAM17 1A2 | 1177 | LRIT3 | 1716 | OR10A 7 | 2255 | SLC18 A3 |
| 100 | ADIPO R2 | 639 | FAM17 1B | 1178 | LRP1 | 1717 | OR10A C1 | 2256 | SLC19 A1 |
| 101 | ADOR A1 | 640 | FAM17 4A | 1179 | LRP1B | 1718 | OR10A D1 | 2257 | SLC19 A2 |
| 102 | ADOR A2A | 641 | FAM17 4B | 1180 | LRP2 | 1719 | OR10A G1 | 2258 | SLC19 A3 |
| 103 | ADOR A2B | 642 | FAM18 7B | 1181 | LRP3 | 1720 | OR10C 1 | 2259 | SLC20 A2 |
| 104 | ADOR A3 | 643 | FAM18 9B | 1182 | LRP4 | 1721 | OR10D 3 | 2260 | SLC22 A1 |
| 105 | ADRA1 A | 644 | FAP | 1183 | LRP5 | 1722 | OR10G 2 | 2261 | SLC22 A2 |
| 106 | ADRA1 B | 645 | FAS | 1184 | LRP6 | 1723 | OR10G 3 | 2262 | SLC22 A3 |
| 107 | ADRA1 D | 646 | FASLG | 1185 | LRP8 | 1724 | OR10G 4 | 2263 | SLC22 A4 |
| 108 | ADRA2 A | 647 | FAT1 | 1186 | LRP10 | 1725 | OR10G 6 | 2264 | SLC22 A5 |
| 109 | ADRA2 B | 648 | FAT2 | 1187 | LRP11 | 1726 | OR10G 7 | 2265 | SLC22 A6 |
| 110 | ADRA2 C | 649 | FAT3 | 1188 | LRP12 | 1727 | OR10G 8 | 2266 | SLC22 A7 |
| 111 | ADRB1 | 650 | FAT4 | 1189 | LRRC3 B | 1728 | OR10G 9 | 2267 | SLC22 A8 |
| 112 | ADRB2 | 651 | FCAM R | 1190 | LRRC4 | 1729 | OR10H 1 | 2268 | SLC22 A9 |
| 113 | ADRB3 | 652 | FCAR | 1191 | LRRC4 B | 1730 | OR10H 2 | 2269 | SLC22 A11 |
| 114 | AGER | 653 | FCER1 A | 1192 | LRRC4 C | 1731 | OR10H 3 | 2270 | SLC22 A12 |
| 115 | AGTR1 | 654 | FCGR1 A | 1193 | LRRC1 5 | 1732 | OR10H 4 | 2271 | SLC22 A13 |
| 116 | AGTR2 | 655 | FCGR1 B | 1194 | LRRC1 9 | 1733 | OR10H 5 | 2272 | SLC22 A14 |
| 117 | AJAP1 | 656 | FCGR2 A | 1195 | LRRC2 4 | 1734 | OR10J 1 | 2273 | SLC22 A15 |
| 118 | ALCA M | 657 | FCGR2 B | 1196 | LRRC2 5 | 1735 | OR10J 3 | 2274 | SLC22 A16 |
| 119 | ALK | 658 | FCGR2 C | 1197 | LRRC3 2 | 1736 | OR10J 4 | 2275 | SLC22 A17 |
| 120 | ALPG | 659 | FCGR3 A | 1198 | LRRC3 7A | 1737 | OR10J 5 | 2276 | SLC22 A23 |
| 121 | ALPI | 660 | FCGR3 B | 1199 | LRRC3 7A2 | 1738 | OR10K 1 | 2277 | SLC22 A25 |
| 122 | ALPL | 661 | FCGR T | 1200 | LRRC3 7A3 | 1739 | OR10K 2 | 2278 | SLC23 A1 |
| 123 | ALPP | 662 | FCRL1 | 1201 | LRRC3 7B | 1740 | OR10P 1 | 2279 | SLC23 A2 |
| 124 | AMHR 2 | 663 | FCRL2 | 1202 | LRRC3 8 | 1741 | OR10Q 1 | 2280 | SLC24 A2 |
| 125 | AMIGO 1 | 664 | FCRL3 | 1203 | LRRC5 2 | 1742 | OR10R 2 | 2281 | SLC24 A3 |
| 126 | AMIGO 2 | 665 | FCRL4 | 1204 | LRRN1 | 1743 | OR10S 1 | 2282 | SLC24 A4 |
| 127 | AMIGO 3 | 666 | FCRL5 | 1205 | LRRN2 | 1744 | OR10T 2 | 2283 | SLC24 A5 |
| 128 | AMN | 667 | FCRL6 | 1206 | LRRN3 | 1745 | OR10V 1 | 2284 | SLC26 A1 |
| 129 | ANKH | 668 | FFAR1 | 1207 | LRRN4 | 1746 | OR10 W1 | 2285 | SLC26 A2 |
| 130 | ANO1 | 669 | FFAR2 | 1208 | LRRN4 CL | 1747 | OR10X 1 | 2286 | SLC26 A3 |
| 131 | ANO2 | 670 | FFAR3 | 1209 | LRRT M2 | 1748 | OR10Z 1 | 2287 | SLC26 A4 |
| 132 | ANO3 | 671 | FFAR4 | 1210 | LRRT M3 | 1749 | OR11A 1 | 2288 | SLC26 A5 |
| 133 | ANO5 | 672 | FGFR1 | 1211 | LRRT M4 | 1750 | OR11G 2 | 2289 | SLC26 A6 |
| 134 | ANO6 | 673 | FGFR2 | 1212 | LRTM1 | 1751 | OR11H 1 | 2290 | SLC26 A8 |
| 135 | ANO7 | 674 | FGFR3 | 1213 | LRTM2 | 1752 | OR11H 2 | 2291 | SLC26 A9 |
| 136 | ANO9 | 675 | FGFR4 | 1214 | LSAMP | 1753 | OR11H 4 | 2292 | SLC28 A1 |
| 137 | ANPEP | 676 | FGFRL 1 | 1215 | LSMEM1 | 1754 | OR11H 6 | 2293 | SLC28 A2 |
| 138 | ANTXR 1 | 677 | FKRP | 1216 | LTB4R | 1755 | OR11H 7 | 2294 | SLC28 A3 |
| 139 | ANTXR 2 | 678 | FLRT1 | 1217 | LTB4R 2 | 1756 | OR11H 12 | 2295 | SLC29 A1 |
| 140 | ANTXR L | 679 | FLRT2 | 1218 | LTBR | 1757 | OR11L 1 | 2296 | SLC29 A2 |
| 141 | AOC3 | 680 | FLRT3 | 1219 | LTK | 1758 | OR12D 2 | 2297 | SLC29 A3 |
| 142 | APCD D1 | 681 | FLT1 | 1220 | LY6D | 1759 | OR12D 3 | 2298 | SLC29 A4 |
| 143 | APLNR | 682 | FLT3 | 1221 | LY6E | 1760 | OR13A 1 | 2299 | SLC30 A1 |
| 144 | APLP1 | 683 | FLT3L G | 1222 | LY6G6 C | 1761 | OR13C 2 | 2300 | SLC31 A1 |
| 145 | APLP2 | 684 | FLT4 | 1223 | LY6G6 D | 1762 | OR13C 3 | 2301 | SLC32 A1 |
| 146 | APP | 685 | FLVCR 1 | 1224 | LY6G6 F | 1763 | OR13C 4 | 2302 | SLC33 A1 |
| 147 | AQP1 | 686 | FLVCR 2 | 1225 | LY6H | 1764 | OR13C 5 | 2303 | SLC34 A1 |
| 148 | AQP2 | 687 | FNDC4 | 1226 | LY6K | 1765 | OR13C 8 | 2304 | SLC34 A2 |
| 149 | AQP4 | 688 | FNDC5 | 1227 | LY9 | 1766 | OR13C 9 | 2305 | SLC34 A3 |
| 150 | AQP5 | 689 | FNDC9 | 1228 | LY75 | 1767 | OR13D 1 | 2306 | SLC35 A5 |
| 151 | AQP8 | 690 | FNDC1 0 | 1229 | LYNX1 | 1768 | OR13F 1 | 2307 | SLC35 F4 |
| 152 | AQP9 | 691 | FOLH1 | 1230 | LYPD1 | 1769 | OR13G 1 | 2308 | SLC36 A1 |
| 153 | AQP10 | 692 | FOLR1 | 1231 | LYPD2 | 1770 | OR13H 1 | 2309 | SLC36 A2 |
| 154 | AREG | 693 | FOLR2 | 1232 | LYPD3 | 1771 | OR13J 1 | 2310 | SLC36 A3 |
| 155 | ARMH 4 | 694 | FPR1 | 1233 | LYPD4 | 1772 | OR14A 2 | 2311 | SLC36 A4 |
| 156 | ART1 | 695 | FPR2 | 1234 | LYPD5 | 1773 | OR14A 16 | 2312 | SLC37 A1 |
| 157 | ART3 | 696 | FPR3 | 1235 | LYPD6 B | 1774 | OR14C 36 | 2313 | SLC37 A2 |
| 158 | ART4 | 697 | FRAS1 | 1236 | LYPD8 | 1775 | OR14I 1 | 2314 | SLC37 A3 |
| 159 | ASGR1 | 698 | FREM2 | 1237 | LYSMD3 | 1776 | OR14J 1 | 2315 | SLC37 A4 |
| 160 | ASGR2 | 699 | FRRS1 | 1238 | LYVE1 | 1777 | OR14K 1 | 2316 | SLC38 A1 |
| 161 | ASIC1 | 700 | FSHR | 1239 | M6PR | 1778 | OR51A 2 | 2317 | SLC38 A2 |
| 162 | ASIC4 | 701 | FURIN | 1240 | MAG | 1779 | OR51A 4 | 2318 | SLC38 A4 |
| 163 | ASIC5 | 702 | FZD1 | 1241 | MALR D1 | 1780 | OR51A 7 | 2319 | SLC38 A5 |
| 164 | ASTN1 | 703 | FZD2 | 1242 | MAMD C4 | 1781 | OR51B 2 | 2320 | SLC38 A8 |
| 165 | ASTN2 | 704 | FZD3 | 1243 | MANS C1 | 1782 | OR51B 4 | 2321 | SLC38 A9 |
| 166 | ATG9A | 705 | FZD4 | 1244 | MANS C4 | 1783 | OR51B 5 | 2322 | SLC38 A11 |
| 167 | ATP1A 1 | 706 | FZD5 | 1245 | MAS1 | 1784 | OR51B 6 | 2323 | SLC39 A2 |
| 168 | ATP1A 2 | 707 | FZD6 | 1246 | MAS1L | 1785 | OR51D 1 | 2324 | SLC39 A4 |
| 169 | ATP1A 3 | 708 | FZD7 | 1247 | MC1R | 1786 | OR51E 1 | 2325 | SLC39 A5 |
| 170 | ATP1A 4 | 709 | FZD8 | 1248 | MC2R | 1787 | OR51E 2 | 2326 | SLC39 A6 |
| 171 | ATP1B 1 | 710 | FZD9 | 1249 | MC3R | 1788 | OR51F 1 | 2327 | SLC39 A8 |
| 172 | ATP1B 2 | 711 | FZD10 | 1250 | MC4R | 1789 | OR51F 2 | 2328 | SLC39 A9 |
| 173 | ATP1B 3 | 712 | GABB R1 | 1251 | MC5R | 1790 | OR51G 1 | 2329 | SLC39 A10 |
| 174 | ATP1B 4 | 713 | GABB R2 | 1252 | MCAM | 1791 | OR51G 2 | 2330 | SLC39 A12 |
| 175 | ATP2B 2 | 714 | GABR A1 | 1253 | MCHR 1 | 1792 | OR51H 1 | 2331 | SLC39 A14 |
| 176 | ATP2B 3 | 715 | GABR A2 | 1254 | MCHR 2 | 1793 | OR51I 1 | 2332 | SLC40 A1 |
| 177 | ATP2B 4 | 716 | GABR A3 | 1255 | MCOL N1 | 1794 | OR51I 2 | 2333 | SLC41 A1 |
| 178 | ATP4B | 717 | GABR A4 | 1256 | MDGA 1 | 1795 | OR51J 1 | 2334 | SLC41 A2 |
| 179 | ATP6V 0A2 | 718 | GABR A5 | 1257 | MDGA 2 | 1796 | OR51L 1 | 2335 | SLC41 A3 |
| 180 | ATP13 A1 | 719 | GABR A6 | 1258 | MEGF 8 | 1797 | OR51 M1 | 2336 | SLC43 A1 |
| 181 | ATP13 A2 | 720 | GABR B1 | 1259 | MEGF 9 | 1798 | OR51Q 1 | 2337 | SLC43 A2 |
| 182 | ATP13 A3 | 721 | GABR B2 | 1260 | MEGF 10 | 1799 | OR51S 1 | 2338 | SLC43 A3 |
| 183 | ATP13 A5 | 722 | GABR B3 | 1261 | MEGF 11 | 1800 | OR51T 1 | 2339 | SLC44 A1 |
| 184 | ATRAI D | 723 | GABR D | 1262 | MELTF | 1801 | OR51V 1 | 2340 | SLC44 A2 |
| 185 | ATRN | 724 | GABR E | 1263 | MEP1A | 1802 | OR52A 1 | 2341 | SLC44 A3 |
| 186 | ATRNL 1 | 725 | GABR G1 | 1264 | MEP1B | 1803 | OR52A 5 | 2342 | SLC44 A4 |
| 187 | AVPR1 A | 726 | GABR G2 | 1265 | MERT K | 1804 | OR52B 2 | 2343 | SLC44 A5 |
| 188 | AVPR1 B | 727 | GABR G3 | 1266 | MET | 1805 | OR52B 4 | 2344 | SLC45 A2 |
| 189 | AVPR2 | 728 | GABR P | 1267 | MFAP3 | 1806 | OR52B 6 | 2345 | SLC45 A4 |
| 190 | AXL | 729 | GABR Q | 1268 | MFAP3 L | 1807 | OR52D 1 | 2346 | SLC46 A1 |
| 191 | BACE1 | 730 | GABR R1 | 1269 | MFRP | 1808 | OR52E 1 | 2347 | SLC46 A2 |
| 192 | BACE2 | 731 | GABR R2 | 1270 | MFSD2A | 1809 | OR52E 2 | 2348 | SLC46 A3 |
| 193 | BAMBI | 732 | GABR R3 | 1271 | MFSD2 B | 1810 | OR52E 4 | 2349 | SLC47 A1 |
| 194 | BCAM | 733 | GALR1 | 1272 | MFSD4 A | 1811 | OR52E 5 | 2350 | SLC49 A3 |
| 195 | BCAN | 734 | GALR2 | 1273 | MFSD4 B | 1812 | OR52E 6 | 2351 | SLC51 A |
| 196 | BDKR B1 | 735 | GALR3 | 1274 | MFSD5 | 1813 | OR52E 8 | 2352 | SLC51 B |
| 197 | BDKR B2 | 736 | GAS1 | 1275 | MFSD6 | 1814 | OR52H 1 | 2353 | SLC52 A1 |
| 198 | BEST2 | 737 | GCGR | 1276 | MFSD6 L | 1815 | OR52I 1 | 2354 | SLC52 A2 |
| 199 | BEST4 | 738 | GDPD 2 | 1277 | MFSD8 | 1816 | OR52I 2 | 2355 | SLC52 A3 |
| 200 | BMPR 1A | 739 | GDPD 5 | 1278 | MFSD1 1 | 1817 | OR52J 3 | 2356 | SLCO1 A2 |
| 201 | BMPR 2 | 740 | GFRA1 | 1279 | MFSD1 2 | 1818 | OR52K 1 | 2357 | SLCO1 B1 |
| 202 | BOC | 741 | GFRA2 | 1280 | MFSD1 4A | 1819 | OR52K 2 | 2358 | SLCO1 B3 |
| 203 | BRS3 | 742 | GFRA3 | 1281 | MICA | 1820 | OR52L 1 | 2359 | SLCO1 B7 |
| 204 | BSG | 743 | GFRA4 | 1282 | MICB | 1821 | OR52 M1 | 2360 | SLCO1 C1 |
| 205 | BST1 | 744 | GFRAL | 1283 | MILR1 | 1822 | OR52N 1 | 2361 | SLCO2 A1 |
| 206 | BST2 | 745 | GFY | 1284 | MIP | 1823 | OR52N 2 | 2362 | SLCO2 B1 |
| 207 | BTC | 746 | GGT1 | 1285 | MLNR | 1824 | OR52N 4 | 2363 | SLCO3 A1 |
| 208 | BTLA | 747 | GGT7 | 1286 | MME | 1825 | OR52N 5 | 2364 | SLCO4 A1 |
| 209 | BTN1A 1 | 748 | GHR | 1287 | MMEL 1 | 1826 | OR52R 1 | 2365 | SLCO4 C1 |
| 210 | BTN2A 1 | 749 | GHRH R | 1288 | MMP1 4 | 1827 | OR52 W1 | 2366 | SLCO5 A1 |
| 211 | BTN2A 2 | 750 | GHSR | 1289 | MMP1 6 | 1828 | OR52Z 1 | 2367 | SLCO6 A1 |
| 212 | BTN3A 1 | 751 | GINM1 | 1290 | MMP1 7 | 1829 | OR56A 1 | 2368 | SLITR K1 |
| 213 | BTN3A 2 | 752 | GIPR | 1291 | MMP2 5 | 1830 | OR56A 3 | 2369 | SLITR K2 |
| 214 | BTN3A 3 | 753 | GJA1 | 1292 | MOG | 1831 | OR56A 4 | 2370 | SLITR K3 |
| 215 | BTNL3 | 754 | GJA3 | 1293 | MOSM O | 1832 | OR56A 5 | 2371 | SLITR K4 |
| 216 | BTNL9 | 755 | GJA4 | 1294 | MPEG 1 | 1833 | OR56B 1 | 2372 | SLITR K5 |
| 217 | BVES | 756 | GJB1 | 1295 | MPIG6 B | 1834 | OR56B 4 | 2373 | SLITR K6 |
| 218 | C1orf1 59 | 757 | GJB2 | 1296 | MPL | 1835 | OSMR | 2374 | SLURP 2 |
| 219 | C3AR1 | 758 | GJB3 | 1297 | MPZ | 1836 | OSTM 1 | 2375 | SMO |
| 220 | C3orf8 0 | 759 | GJB4 | 1298 | MPZL1 | 1837 | OTOA | 2376 | SORC S1 |
| 221 | C5AR1 | 760 | GJB5 | 1299 | MPZL2 | 1838 | OTOP1 | 2377 | SORC S2 |
| 222 | C5AR2 | 761 | GJB6 | 1300 | MPZL3 | 1839 | OTOP2 | 2378 | SORC S3 |
| 223 | C5orf1 5 | 762 | GJB7 | 1301 | MR1 | 1840 | OXER1 | 2379 | SORL1 |
| 224 | C11orf 24 | 763 | GJC2 | 1302 | MRC1 | 1841 | OXGR 1 | 2380 | SORT1 |
| 225 | C11orf 87 | 764 | GJC3 | 1303 | MRC2 | 1842 | OXTR | 2381 | SPACA1 |
| 226 | C14orf 132 | 765 | GJD2 | 1304 | MRGP RD | 1843 | P2RX1 | 2382 | SPACA 4 |
| 227 | C19orf 18 | 766 | GLDN | 1305 | MRGP RE | 1844 | P2RX2 | 2383 | SPAM1 |
| 228 | C19orf 38 | 767 | GLIPR 1 | 1306 | MRGP RF | 1845 | P2RX3 | 2384 | SPINT 2 |
| 229 | CA4 | 768 | GLMP | 1307 | MRGP RG | 1846 | P2RX4 | 2385 | SPN |
| 230 | CA12 | 769 | GLP1R | 1308 | MRGP RX1 | 1847 | P2RX6 | 2386 | SPNS2 |
| 231 | CA14 | 770 | GLP2R | 1309 | MRGP RX2 | 1848 | P2RX7 | 2387 | SPNS3 |
| 232 | CACH D1 | 771 | GLRA1 | 1310 | MRGP RX3 | 1849 | P2RY1 | 2388 | SPPL2 A |
| 233 | CACN A1C | 772 | GLRA2 | 1311 | MRGP RX4 | 1850 | P2RY2 | 2389 | SPPL2 B |
| 234 | CACN A1G | 773 | GLRA3 | 1312 | MRVI1 | 1851 | P2RY4 | 2390 | SPPL2 C |
| 235 | CACN A1I | 774 | GLRA4 | 1313 | MS4A1 | 1852 | P2RY6 | 2391 | SPRN |
| 236 | CACN G1 | 775 | GLRB | 1314 | MS4A6A | 1853 | P2RY8 | 2392 | SSPN |
| 237 | CACN G2 | 776 | GML | 1315 | MS4A1 5 | 1854 | P2RY1 0 | 2393 | SSR1 |
| 238 | CACN G3 | 777 | GNRH R | 1316 | MSLN | 1855 | P2RY1 1 | 2394 | SSTR1 |
| 239 | CACN G4 | 778 | GP1BA | 1317 | MSR1 | 1856 | P2RY1 2 | 2395 | SSTR2 |
| 240 | CACN G5 | 779 | GP1BB | 1318 | MST1R | 1857 | P2RY1 3 | 2396 | SSTR3 |
| 241 | CACN G6 | 780 | GP2 | 1319 | MTNR 1A | 1858 | P2RY1 4 | 2397 | SSTR4 |
| 242 | CACN G7 | 781 | GP5 | 1320 | MTNR 1B | 1859 | PAM | 2398 | SSTR5 |
| 243 | CACN G8 | 782 | GP6 | 1321 | MUC1 | 1860 | PANX1 | 2399 | STAB1 |
| 244 | CADM 1 | 783 | GPA33 | 1322 | MUC3 A | 1861 | PANX2 | 2400 | STAB2 |
| 245 | CADM 2 | 784 | GPBA R1 | 1323 | MUC3 B | 1862 | PARM1 | 2401 | STEAP 4 |
| 246 | CADM 3 | 785 | GPC1 | 1324 | MUC4 | 1863 | PCDH1 | 2402 | STIM1 |
| 247 | CADM 4 | 786 | GPC2 | 1325 | MUC12 | 1864 | PCDH7 | 2403 | STIMATE |
| 248 | CALCR | 787 | GPC3 | 1326 | MUC13 | 1865 | PCDH8 | 2404 | STS |
| 249 | CALCR L | 788 | GPC4 | 1327 | MUC15 | 1866 | PCDH9 | 2405 | STT3B |
| 250 | CALH M2 | 789 | GPC5 | 1328 | MUC16 | 1867 | PCDH1 0 | 2406 | SUCN R1 |
| 251 | CALH M5 | 790 | GPC6 | 1329 | MUC17 | 1868 | PCDH1 1X | 2407 | SUCO |
| 252 | CALY | 791 | GPER1 | 1330 | MUC21 | 1869 | PCDH1 1Y | 2408 | SUSD1 |
| 253 | CASD1 | 792 | GPIHB P1 | 1331 | MUC22 | 1870 | PCDH1 2 | 2409 | SUSD2 |
| 254 | CASR | 793 | GPM6 A | 1332 | MUCL3 | 1871 | PCDH1 5 | 2410 | SUSD3 |
| 255 | CATSP ERD | 794 | GPM6 B | 1333 | MUSK | 1872 | PCDH1 7 | 2411 | SUSD4 |
| 256 | CATSP ERE | 795 | GPNM B | 1334 | MXRA 8 | 1873 | PCDH1 8 | 2412 | SUSD5 |
| 257 | CATSP ERG | 796 | GPR1 | 1335 | MYAD M | 1874 | PCDH1 9 | 2413 | SUSD6 |
| 258 | CCKA R | 797 | GPR3 | 1336 | MYAD ML2 | 1875 | PCDH2 0 | 2414 | SV2A |
| 259 | CCKB R | 798 | GPR4 | 1337 | MYOF | 1876 | PCNX1 | 2415 | SV2B |
| 260 | CCR1 | 799 | GPR6 | 1338 | NAALA DL1 | 1877 | PCNX2 | 2416 | SV2C |
| 261 | CCR2 | 800 | GPR12 | 1339 | NAGP A | 1878 | PCSK5 | 2417 | SVOPL |
| 262 | CCR3 | 801 | GPR15 | 1340 | NALCN | 1879 | PDCD1 | 2418 | SYNP R |
| 263 | CCR4 | 802 | GPR17 | 1341 | NCAM 1 | 1880 | PDCD1 LG2 | 2419 | SYP |
| 264 | CCR5 | 803 | GPR18 | 1342 | NCAM 2 | 1881 | PDGF RA | 2420 | SYPL1 |
| 265 | CCR6 | 804 | GPR19 | 1343 | NCMA P | 1882 | PDGF RB | 2421 | TAAR1 |
| 266 | CCR7 | 805 | GPR20 | 1344 | NCR1 | 1883 | PEAR1 | 2422 | TAAR2 |
| 267 | CCR8 | 806 | GPR21 | 1345 | NCR2 | 1884 | PECA M1 | 2423 | TAAR5 |
| 268 | CCR9 | 807 | GPR22 | 1346 | NCR3 | 1885 | PGAP1 | 2424 | TAAR6 |
| 269 | CCR10 | 808 | GPR25 | 1347 | NCR3L G1 | 1886 | PHEX | 2425 | TAAR8 |
| 270 | CCRL2 | 809 | GPR26 | 1348 | NCST N | 1887 | PI16 | 2426 | TAAR9 |
| 271 | CD1A | 810 | GPR27 | 1349 | NECTI N1 | 1888 | PIEZO 1 | 2427 | TACR1 |
| 272 | CD1B | 811 | GPR31 | 1350 | NECTI N2 | 1889 | PIEZO 2 | 2428 | TACR2 |
| 273 | CD1C | 812 | GPR32 | 1351 | NECTI N3 | 1890 | PIGO | 2429 | TACR3 |
| 274 | CD1D | 813 | GPR33 | 1352 | NECTI N4 | 1891 | PIGR | 2430 | TACST D2 |
| 275 | CD1E | 814 | GPR34 | 1353 | NEGR 1 | 1892 | PIGT | 2431 | TARM1 |
| 276 | CD2 | 815 | GPR35 | 1354 | NEMP 1 | 1893 | PIK3IP 1 | 2432 | TAS1R 1 |
| 277 | CD3D | 816 | GPR37 | 1355 | NEO1 | 1894 | PILRA | 2433 | TAS1R 2 |
| 278 | CD3G | 817 | GPR37 L1 | 1356 | NETO1 | 1895 | PILRB | 2434 | TAS1R 3 |
| 279 | CD4 | 818 | GPR39 | 1357 | NETO2 | 1896 | PKD1 | 2435 | TAS2R 1 |
| 280 | CD5 | 819 | GPR42 | 1358 | NFAM1 | 1897 | PKD1L 1 | 2436 | TAS2R 3 |
| 281 | CD6 | 820 | GPR45 | 1359 | NFASC | 1898 | PKD1L 2 | 2437 | TAS2R 4 |
| 282 | CD7 | 821 | GPR50 | 1360 | NGFR | 1899 | PKD1L 3 | 2438 | TAS2R 7 |
| 283 | CD8A | 822 | GPR52 | 1361 | NIPAL1 | 1900 | PKD2 | 2439 | TAS2R 8 |
| 284 | CD8B | 823 | GPR55 | 1362 | NIPAL2 | 1901 | PKD2L 1 | 2440 | TAS2R 9 |
| 285 | CD9 | 824 | GPR61 | 1363 | NIPAL3 | 1902 | PKDR EJ | 2441 | TAS2R 10 |
| 286 | CD14 | 825 | GPR62 | 1364 | NIPAL4 | 1903 | PKHD1 | 2442 | TAS2R 14 |
| 287 | CD19 | 826 | GPR63 | 1365 | NKAIN 1 | 1904 | PKHD1 L1 | 2443 | TAS2R 16 |
| 288 | CD22 | 827 | GPR65 | 1366 | NKAIN 2 | 1905 | PLA2R 1 | 2444 | TAS2R 19 |
| 289 | CD24 | 828 | GPR68 | 1367 | NKAIN 3 | 1906 | PLAUR | 2445 | TAS2R 20 |
| 290 | CD27 | 829 | GPR75 | 1368 | NLGN1 | 1907 | PLB1 | 2446 | TAS2R 30 |
| 291 | CD28 | 830 | GPR78 | 1369 | NLGN2 | 1908 | PLD5 | 2447 | TAS2R 38 |
| 292 | CD33 | 831 | GPR82 | 1370 | NLGN3 | 1909 | PLET1 | 2448 | TAS2R 39 |
| 293 | CD34 | 832 | GPR83 | 1371 | NLGN4 X | 1910 | PLP1 | 2449 | TAS2R 46 |
| 294 | CD36 | 833 | GPR84 | 1372 | NLGN4 Y | 1911 | PLPP1 | 2450 | TBXA2 R |
| 295 | CD37 | 834 | GPR85 | 1373 | NMBR | 1912 | PLPP2 | 2451 | TCIRG 1 |
| 296 | CD38 | 835 | GPR87 | 1374 | NMUR 1 | 1913 | PLPP3 | 2452 | TCTN2 |
| 297 | CD40 | 836 | GPR88 | 1375 | NMUR 2 | 1914 | PLPPR 1 | 2453 | TCTN3 |
| 298 | CD40L G | 837 | GPR10 1 | 1376 | NOTC H1 | 1915 | PLPPR 4 | 2454 | TDGF1 |
| 299 | CD44 | 838 | GPR10 7 | 1377 | NOTC H2 | 1916 | PLPPR 5 | 2455 | TECTA |
| 300 | CD46 | 839 | GPR10 8 | 1378 | NOTC H3 | 1917 | PLVAP | 2456 | TECTB |
| 301 | CD47 | 840 | GPR11 9 | 1379 | NOTC H4 | 1918 | PLXDC 1 | 2457 | TEK |
| 302 | CD48 | 841 | GPR13 2 | 1380 | NOX4 | 1919 | PLXDC 2 | 2458 | TENM1 |
| 303 | CD52 | 842 | GPR13 7 | 1381 | NPBW R1 | 1920 | PLXNA 1 | 2459 | TENM2 |
| 304 | CD53 | 843 | GPR13 7B | 1382 | NPBW R2 | 1921 | PLXNA 2 | 2460 | TENM3 |
| 305 | CD55 | 844 | GPR13 7C | 1383 | NPC1 | 1922 | PLXNA 3 | 2461 | TENM4 |
| 306 | CD58 | 845 | GPR13 9 | 1384 | NPC1L 1 | 1923 | PLXNA 4 | 2462 | TEX10 1 |
| 307 | CD59 | 846 | GPR14 1 | 1385 | NPFFR 1 | 1924 | PLXNB 1 | 2463 | TFPI |
| 308 | CD63 | 847 | GPR14 2 | 1386 | NPFFR 2 | 1925 | PLXNB 2 | 2464 | TGFA |
| 309 | CD68 | 848 | GPR14 3 | 1387 | NPHS1 | 1926 | PLXNB 3 | 2465 | TGFBR 1 |
| 310 | CD69 | 849 | GPR14 6 | 1388 | NPR1 | 1927 | PLXNC 1 | 2466 | TGFBR 2 |
| 311 | CD70 | 850 | GPR14 8 | 1389 | NPR2 | 1928 | PLXND 1 | 2467 | TGFBR 3 |
| 312 | CD74 | 851 | GPR14 9 | 1390 | NPR3 | 1929 | PMEL | 2468 | TGOL N2 |
| 313 | CD79A | 852 | GPR15 0 | 1391 | NPSR1 | 1930 | PMEP A1 | 2469 | THBD |
| 314 | CD79B | 853 | GPR15 1 | 1392 | NPTN | 1931 | PODXL | 2470 | THSD1 |
| 315 | CD80 | 854 | GPR15 2 | 1393 | NPY1R | 1932 | PODXL 2 | 2471 | THSD7 A |
| 316 | CD82 | 855 | GPR15 3 | 1394 | NPY2R | 1933 | PQLC2 | 2472 | THSD7 B |
| 317 | CD83 | 856 | GPR15 5 | 1395 | NPY4R | 1934 | PRIMA 1 | 2473 | THY1 |
| 318 | CD84 | 857 | GPR15 6 | 1396 | NPY5R | 1935 | PRLHR | 2474 | TIE1 |
| 319 | CD86 | 858 | GPR15 7 | 1397 | NRCA M | 1936 | PRLR | 2475 | TIGIT |
| 320 | CD93 | 859 | GPR15 8 | 1398 | NRG1 | 1937 | PRND | 2476 | TIMD4 |
| 321 | CD96 | 860 | GPR16 0 | 1399 | NRG2 | 1938 | PRNP | 2477 | TLR1 |
| 322 | CD101 | 861 | GPR16 1 | 1400 | NRG4 | 1939 | PROC R | 2478 | TLR2 |
| 323 | CD109 | 862 | GPR16 2 | 1401 | NRN1 | 1940 | PROK R1 | 2479 | TLR3 |
| 324 | CD151 | 863 | GPR17 1 | 1402 | NRN1L | 1941 | PROK R2 | 2480 | TLR4 |
| 325 | CD160 | 864 | GPR17 3 | 1403 | NRP1 | 1942 | PROM 1 | 2481 | TLR5 |
| 326 | CD163 | 865 | GPR17 4 | 1404 | NRP2 | 1943 | PROM 2 | 2482 | TLR6 |
| 327 | CD163 L1 | 866 | GPR17 6 | 1405 | NRRO S | 1944 | PRPH2 | 2483 | TLR7 |
| 328 | CD164 | 867 | GPR17 9 | 1406 | NRXN1 | 1945 | PRRT3 | 2484 | TLR8 |
| 329 | CD164 L2 | 868 | GPR18 0 | 1407 | NRXN2 | 1946 | PRSS8 | 2485 | TLR9 |
| 330 | CD177 | 869 | GPR18 2 | 1408 | NRXN3 | 1947 | PRSS2 1 | 2486 | TLR10 |
| 331 | CD180 | 870 | GPR18 3 | 1409 | NT5E | 1948 | PRSS4 1 | 2487 | TM4SF 1 |
| 332 | CD200 | 871 | GPRC 5A | 1410 | NTM | 1949 | PRTG | 2488 | TM4SF 4 |
| 333 | CD200 R1 | 872 | GPRC 5B | 1411 | NTNG1 | 1950 | PSCA | 2489 | TM4SF 5 |
| 334 | CD200 R1L | 873 | GPRC 5C | 1412 | NTNG2 | 1951 | PSEN1 | 2490 | TM4SF 18 |
| 335 | CD226 | 874 | GPRC 5D | 1413 | NTRK1 | 1952 | PSEN2 | 2491 | TM4SF 20 |
| 336 | CD244 | 875 | GPRC 6A | 1414 | NTRK2 | 1953 | PTAFR | 2492 | TM7SF 3 |
| 337 | CD248 | 876 | GRAM D1B | 1415 | NTRK3 | 1954 | PTCH1 | 2493 | TM9SF 1 |
| 338 | CD274 | 877 | GRIA1 | 1416 | NTSR1 | 1955 | PTCH D1 | 2494 | TM9SF 2 |
| 339 | CD276 | 878 | GRIA2 | 1417 | NTSR2 | 1956 | PTCH D3 | 2495 | TM9SF 3 |
| 340 | CD300 A | 879 | GRIA3 | 1418 | NUP21 0 | 1957 | PTCH D4 | 2496 | TM9SF 4 |
| 341 | CD300 C | 880 | GRIA4 | 1419 | NUP21 0L | 1958 | PTCRA | 2497 | TMC7 |
| 342 | CD300 E | 881 | GRID1 | 1420 | OLR1 | 1959 | PTGD R | 2498 | TMCO 3 |
| 343 | CD300 LD | 882 | GRID2 | 1421 | OMG | 1960 | PTGD R2 | 2499 | TMED7 |
| 344 | CD300 LF | 883 | GRIK1 | 1422 | OPALI N | 1961 | PTGE R1 | 2500 | TMEFF 1 |
| 345 | CD300 LG | 884 | GRIK2 | 1423 | OPCM L | 1962 | PTGE R2 | 2501 | TMEFF 2 |
| 346 | CD302 | 885 | GRIK3 | 1424 | OPN1L W | 1963 | PTGE R3 | 2502 | TMEM 8A |
| 347 | CD320 | 886 | GRIK4 | 1425 | OPN1 MW | 1964 | PTGE R4 | 2503 | TMEM 8B |
| 348 | CDCP1 | 887 | GRIK5 | 1426 | OPN1S W | 1965 | PTGFR | 2504 | TMEM 9 |
| 349 | CDH1 | 888 | GRIN1 | 1427 | OPN3 | 1966 | PTGFR N | 2505 | TMEM 9B |
| 350 | CDH2 | 889 | GRIN2 A | 1428 | OPN4 | 1967 | PTGIR | 2506 | TMEM 25 |
| 351 | CDH3 | 890 | GRIN2 B | 1429 | OPN5 | 1968 | PTH1R | 2507 | TMEM 26 |
| 352 | CDH4 | 891 | GRIN2 C | 1430 | OPRD 1 | 1969 | PTH2R | 2508 | TMEM 30A |
| 353 | CDH5 | 892 | GRIN2 D | 1431 | OPRK1 | 1970 | PTK7 | 2509 | TMEM 37 |
| 354 | CDH6 | 893 | GRIN3 A | 1432 | OPRL1 | 1971 | PTPRA | 2510 | TMEM 62 |
| 355 | CDH7 | 894 | GRIN3 B | 1433 | OPRM 1 | 1972 | PTPRB | 2511 | TMEM 63A |
| 356 | CDH8 | 895 | GRM1 | 1434 | OR1A1 | 1973 | PTPRC | 2512 | TMEM 63B |
| 357 | CDH9 | 896 | GRM2 | 1435 | OR1A2 | 1974 | PTPRD | 2513 | TMEM 63C |
| 358 | CDH10 | 897 | GRM3 | 1436 | OR1B1 | 1975 | PTPRF | 2514 | TMEM 67 |
| 359 | CDH11 | 898 | GRM4 | 1437 | OR1C1 | 1976 | PTPR G | 2515 | TMEM 87A |
| 360 | CDH12 | 899 | GRM5 | 1438 | OR1D2 | 1977 | PTPRH | 2516 | TMEM 87B |
| 361 | CDH13 | 900 | GRM6 | 1439 | OR1D4 | 1978 | PTPRJ | 2517 | TMEM 95 |
| 362 | CDH15 | 901 | GRM7 | 1440 | OR1D5 | 1979 | PTPRK | 2518 | TMEM 104 |
| 363 | CDH16 | 902 | GRM8 | 1441 | OR1E1 | 1980 | PTPR M | 2519 | TMEM 106A |
| 364 | CDH17 | 903 | GRPR | 1442 | OR1E2 | 1981 | PTPRN | 2520 | TMEM 106B |
| 365 | CDH18 | 904 | GSG1 | 1443 | OR1E3 | 1982 | PTPRN 2 | 2521 | TMEM 108 |
| 366 | CDH19 | 905 | GSG1L | 1444 | OR1F1 | 1983 | PTPR O | 2522 | TMEM 114 |
| 367 | CDH20 | 906 | GSG1L 2 | 1445 | OR1G1 | 1984 | PTPR Q | 2523 | TMEM 116 |
| 368 | CDH22 | 907 | GUCY 2C | 1446 | OR1I1 | 1985 | PTPRR | 2524 | TMEM 123 |
| 369 | CDH23 | 908 | GYPA | 1447 | OR1J1 | 1986 | PTPRS | 2525 | TMEM 131L |
| 370 | CDH24 | 909 | GYPC | 1448 | OR1J2 | 1987 | PTPRT | 2526 | TMEM 132A |
| 371 | CDH26 | 910 | HAVC R1 | 1449 | OR1J4 | 1988 | PTPRU | 2527 | TMEM 132B |
| 372 | CDHR 1 | 911 | HAVC R2 | 1450 | OR1K1 | 1989 | PTPRZ 1 | 2528 | TMEM 132C |
| 373 | CDHR 2 | 912 | HBEG F | 1451 | OR1L1 | 1990 | PTTG1 IP | 2529 | TMEM 132D |
| 374 | CDHR 3 | 913 | HCAR1 | 1452 | OR1L3 | 1991 | PVR | 2530 | TMEM 132E |
| 375 | CDHR 4 | 914 | HCAR2 | 1453 | OR1L4 | 1992 | QRFP R | 2531 | TMEM 140 |
| 376 | CDHR 5 | 915 | HCAR3 | 1454 | OR1L6 | 1993 | QSOX 1 | 2532 | TMEM 145 |
| 377 | CDON | 916 | HCRT R1 | 1455 | OR1L8 | 1994 | QSOX 2 | 2533 | TMEM 150A |
| 378 | CEAC AM1 | 917 | HCRT R2 | 1456 | OR1M 1 | 1995 | RAET1 E | 2534 | TMEM 150B |
| 379 | CEAC AM3 | 918 | HEG1 | 1457 | OR1N1 | 1996 | RAET1 G | 2535 | TMEM 154 |
| 380 | CEAC AM4 | 919 | HEPAC AM | 1458 | OR1N2 | 1997 | RAET1 L | 2536 | TMEM 158 |
| 381 | CEAC AM5 | 920 | HEPAC AM2 | 1459 | OR1P1 | 1998 | RAMP 2 | 2537 | TMEM 161A |
| 382 | CEAC AM6 | 921 | HEPH | 1460 | OR1Q1 | 1999 | RAMP 3 | 2538 | TMEM 171 |
| 383 | CEAC AM7 | 922 | HEPHL 1 | 1461 | OR1S1 | 2000 | RECK | 2539 | TMEM 178A |
| 384 | CEAC AM8 | 923 | HFE | 1462 | OR1S2 | 2001 | RELL1 | 2540 | TMEM 178B |
| 385 | CEAC AM19 | 924 | HHLA2 | 1463 | OR2A1 | 2002 | RELT | 2541 | TMEM 179B |
| 386 | CEAC AM20 | 925 | HJV | 1464 | OR2A2 | 2003 | RET | 2542 | TMEM 182 |
| 387 | CEAC AM21 | 926 | HLA-A | 1465 | OR2A4 | 2004 | RGMA | 2543 | TMEM 184A |
| 388 | CELSR 1 | 927 | HLA-B | 1466 | OR2A5 | 2005 | RGMB | 2544 | TMEM 204 |
| 389 | CELSR 2 | 928 | HLA-C | 1467 | OR2A7 | 2006 | RGR | 2545 | TMEM 211 |
| 390 | CELSR 3 | 929 | HLA-DMA | 1468 | OR2A1 2 | 2007 | RHAG | 2546 | TMEM 213 |
| 391 | CFC1 | 930 | HLA-DMB | 1469 | OR2A1 4 | 2008 | RHBD F2 | 2547 | TMEM 217 |
| 392 | CHL1 | 931 | HLA-DOA | 1470 | OR2A2 5 | 2009 | RHBDL 2 | 2548 | TMEM 219 |
| 393 | CHOD L | 932 | HLA-DOB | 1471 | OR2A4 2 | 2010 | RHCG | 2549 | TMEM 225 |
| 394 | CHPT1 | 933 | HLA-DPA1 | 1472 | OR2AE 1 | 2011 | RHO | 2550 | TMEM 231 |
| 395 | CHRM 1 | 934 | HLA-DPB1 | 1473 | OR2A G1 | 2012 | RNF13 | 2551 | TMEM 235 |
| 396 | CHRM 2 | 935 | HLA-DQA1 | 1474 | OR2A G2 | 2013 | RNF43 | 2552 | TMEM 245 |
| 397 | CHRM 3 | 936 | HLA-DQA2 | 1475 | OR2AJ 1 | 2014 | RNF12 8 | 2553 | TMEM 255A |
| 398 | CHRM 4 | 937 | HLA-DQB1 | 1476 | OR2AK 2 | 2015 | RNF13 0 | 2554 | TMEM 255B |
| 399 | CHRM 5 | 938 | HLA-DQB2 | 1477 | OR2AP 1 | 2016 | RNF14 9 | 2555 | TMIGD 1 |
| 400 | CHRN A1 | 939 | HLA-DRA | 1478 | OR2AT 4 | 2017 | RNF15 0 | 2556 | TMIGD 2 |
| 401 | CHRN A2 | 940 | HLA-DRB1 | 1479 | OR2B2 | 2018 | RNF16 7 | 2557 | TMIGD 3 |
| 402 | CHRN A3 | 941 | HLA-DRB5 | 1480 | OR2B3 | 2019 | RNFT1 | 2558 | TMPR SS5 |
| 403 | CHRN A4 | 942 | HLA-E | 1481 | OR2B6 | 2020 | ROBO 1 | 2559 | TMPR SS6 |
| 404 | CHRN A5 | 943 | HLA-F | 1482 | OR2B1 1 | 2021 | ROBO 2 | 2560 | TMPR SS11B |
| 405 | CHRN A6 | 944 | HLA-G | 1483 | OR2C1 | 2022 | ROBO 3 | 2561 | TMPR SS11D |
| 406 | CHRN A7 | 945 | HM13 | 1484 | OR2C3 | 2023 | ROR1 | 2562 | TMPR SS11E |
| 407 | CHRN A9 | 946 | HRH1 | 1485 | OR2D2 | 2024 | ROR2 | 2563 | TMPR SS13 |
| 408 | CHRN A10 | 947 | HRH2 | 1486 | OR2D3 | 2025 | ROS1 | 2564 | TMPR SS15 |
| 409 | CHRN B1 | 948 | HRH3 | 1487 | OR2F1 | 2026 | RPN1 | 2565 | TMX3 |
| 410 | CHRN B2 | 949 | HRH4 | 1488 | OR2F2 | 2027 | RPRM | 2566 | TMX4 |
| 411 | CHRN B3 | 950 | HTR1A | 1489 | OR2G2 | 2028 | RPRM L | 2567 | TNFRS F1A |
| 412 | CHRN B4 | 951 | HTR1B | 1490 | OR2G3 | 2029 | RRH | 2568 | TNFRS F1B |
| 413 | CHRN D | 952 | HTR1D | 1491 | OR2G6 | 2030 | RTN4R | 2569 | TNFRS F4 |
| 414 | CHRN E | 953 | HTR1E | 1492 | OR2H1 | 2031 | RTN4R L1 | 2570 | TNFRS F8 |
| 415 | CHRN G | 954 | HTR1F | 1493 | OR2H2 | 2032 | RTN4R L2 | 2571 | TNFRS F9 |
| 416 | CLCA2 | 955 | HTR2A | 1494 | OR2J1 | 2033 | RXFP1 | 2572 | TNFRS F10A |
| 417 | CLCA4 | 956 | HTR2B | 1495 | OR2J2 | 2034 | RXFP2 | 2573 | TNFRS F10C |
| 418 | CLCNK B | 957 | HTR2C | 1496 | OR2J3 | 2035 | RXFP3 | 2574 | TNFRS F10D |
| 419 | CLDN1 | 958 | HTR3A | 1497 | OR2K2 | 2036 | RXFP4 | 2575 | TNFRS F11A |
| 420 | CLDN2 | 959 | HTR3B | 1498 | OR2L2 | 2037 | RYK | 2576 | TNFRS F13B |
| 421 | CLDN3 | 960 | HTR3C | 1499 | OR2L3 | 2038 | S1PR1 | 2577 | TNFRS F14 |
| 422 | CLDN4 | 961 | HTR3D | 1500 | OR2L5 | 2039 | S1PR2 | 2578 | TNFRS F17 |
| 423 | CLDN6 | 962 | HTR3E | 1501 | OR2L8 | 2040 | S1PR3 | 2579 | TNFRS F18 |
| 424 | CLDN7 | 963 | HTR4 | 1502 | OR2L1 3 | 2041 | S1PR4 | 2580 | TNFRS F19 |
| 425 | CLDN8 | 964 | HTR5A | 1503 | OR2M 2 | 2042 | S1PR5 | 2581 | TNFRS F21 |
| 426 | CLDN9 | 965 | HTR6 | 1504 | OR2M 3 | 2043 | SCAP | 2582 | TNFRS F25 |
| 427 | CLDN1 0 | 966 | HTR7 | 1505 | OR2M 4 | 2044 | SCAR A5 | 2583 | TNFSF 4 |
| 428 | CLDN1 1 | 967 | HYAL2 | 1506 | OR2M 5 | 2045 | SCAR B1 | 2584 | TNFSF 8 |
| 429 | CLDN1 2 | 968 | ICAM1 | 1507 | OR2M 7 | 2046 | SCAR B2 | 2585 | TNFSF 11 |
| 430 | CLDN1 5 | 969 | ICAM2 | 1508 | OR2S2 | 2047 | SCARF 1 | 2586 | TNFSF 13B |
| 431 | CLDN1 6 | 970 | ICAM3 | 1509 | OR2T1 | 2048 | SCARF 2 | 2587 | TNFSF 15 |
| 432 | CLDN1 8 | 971 | ICAM4 | 1510 | OR2T2 | 2049 | SCN1A | 2588 | TNFSF 18 |
| 433 | CLDN1 9 | 972 | ICAM5 | 1511 | OR2T3 | 2050 | SCN1B | 2589 | TP53I1 3 |
| 434 | CLDN2 0 | 973 | ICOS | 1512 | OR2T4 | 2051 | SCN2A | 2590 | TPBG |
| 435 | CLDN2 2 | 974 | ICOSL G | 1513 | OR2T5 | 2052 | SCN2B | 2591 | TPBGL |
| 436 | CLDN2 4 | 975 | IFNAR 1 | 1514 | OR2T6 | 2053 | SCN3A | 2592 | TPCN1 |
| 437 | CLDN D1 | 976 | IFNAR 2 | 1515 | OR2T7 | 2054 | SCN3B | 2593 | TPO |
| 438 | CLEC1 A | 977 | IFNGR 1 | 1516 | OR2T8 | 2055 | SCN4A | 2594 | TPRA1 |
| 439 | CLEC1 B | 978 | IFNGR 2 | 1517 | OR2T1 0 | 2056 | SCN4B | 2595 | TPSG1 |
| 440 | CLEC2 D | 979 | IFNLR 1 | 1518 | OR2T1 1 | 2057 | SCN5A | 2596 | TRABD 2A |
| 441 | CLEC4 G | 980 | IGDCC 3 | 1519 | OR2T1 2 | 2058 | SCN7A | 2597 | TRABD 2B |
| 442 | CLEC4 M | 981 | IGDCC 4 | 1520 | OR2T2 7 | 2059 | SCN8A | 2598 | TRAT1 |
| 443 | CLEC5 A | 982 | IGF1R | 1521 | OR2T2 9 | 2060 | SCN9A | 2599 | TREH |
| 444 | CLEC7 A | 983 | IGF2R | 1522 | OR2T3 3 | 2061 | SCN10 A | 2600 | TREM1 |
| 445 | CLEC9 A | 984 | IGFLR 1 | 1523 | OR2T3 4 | 2062 | SCN11 A | 2601 | TREM2 |
| 446 | CLEC1 2A | 985 | IGSF1 | 1524 | OR2T3 5 | 2063 | SCNN1 A | 2602 | TREML 2 |
| 447 | CLEC1 2B | 986 | IGSF3 | 1525 | OR2V1 | 2064 | SCNN1 B | 2603 | TRHD E |
| 448 | CLEC1 4A | 987 | IGSF5 | 1526 | OR2V2 | 2065 | SCNN1 D | 2604 | TRHR |
| 449 | CLEC1 7A | 988 | IGSF6 | 1527 | OR2W 1 | 2066 | SCNN1 G | 2605 | TRIL |
| 450 | CLMP | 989 | IGSF8 | 1528 | OR2W 3 | 2067 | SCTR | 2606 | TRPV2 |
| 451 | CLN3 | 990 | IGSF9 | 1529 | OR2Y1 | 2068 | SDC1 | 2607 | TRPV4 |
| 452 | CLRN1 | 991 | IGSF9 B | 1530 | OR2Z1 | 2069 | SDC2 | 2608 | TRPV5 |
| 453 | CLRN2 | 992 | IGSF11 | 1531 | OR3A1 | 2070 | SDK1 | 2609 | TRPV6 |
| 454 | CLSTN 1 | 993 | IL1R1 | 1532 | OR3A2 | 2071 | SDK2 | 2610 | TSHR |
| 455 | CLSTN 2 | 994 | IL1R2 | 1533 | OR3A3 | 2072 | SECT M1 | 2611 | TSPAN 1 |
| 456 | CLSTN 3 | 995 | IL1RA P | 1534 | OR4A5 | 2073 | SELE | 2612 | TSPAN 2 |
| 457 | CLTRN | 996 | IL1RA PL1 | 1535 | OR4A8 | 2074 | SELL | 2613 | TSPAN 3 |
| 458 | CMKL R1 | 997 | IL1RA PL2 | 1536 | OR4A1 5 | 2075 | SELP | 2614 | TSPAN 4 |
| 459 | CNNM 2 | 998 | IL1RL1 | 1537 | OR4A1 6 | 2076 | SELPL G | 2615 | TSPAN 5 |
| 460 | CNNM 3 | 999 | IL1RL2 | 1538 | OR4A4 7 | 2077 | SEMA4 A | 2616 | TSPAN 6 |
| 461 | CNNM 4 | 1000 | IL2RA | 1539 | OR4B1 | 2078 | SEMA4 B | 2617 | TSPAN 7 |
| 462 | CNR1 | 1001 | IL2RB | 1540 | OR4C3 | 2079 | SEMA4 C | 2618 | TSPAN 8 |
| 463 | CNR2 | 1002 | IL2RG | 1541 | OR4C5 | 2080 | SEMA4 D | 2619 | TSPAN 9 |
| 464 | CNTFR | 1003 | IL3RA | 1542 | OR4C6 | 2081 | SEMA4 F | 2620 | TSPAN 11 |
| 465 | CNTN1 | 1004 | IL4R | 1543 | OR4C1 1 | 2082 | SEMA4 G | 2621 | TSPAN 13 |
| 466 | CNTN2 | 1005 | IL5RA | 1544 | OR4C1 2 | 2083 | SEMA5 A | 2622 | TSPAN 14 |
| 467 | CNTN3 | 1006 | IL6R | 1545 | OR4C1 3 | 2084 | SEMA5 B | 2623 | TSPAN 15 |
| 468 | CNTN4 | 1007 | IL6ST | 1546 | OR4C1 5 | 2085 | SEMA6 A | 2624 | TSPAN 17 |
| 469 | CNTN5 | 1008 | IL7R | 1547 | OR4C1 6 | 2086 | SEMA6 B | 2625 | TSPAN 18 |
| 470 | CNTN6 | 1009 | IL9R | 1548 | OR4C4 5 | 2087 | SEMA6 C | 2626 | TSPAN 31 |
| 471 | CNTN AP1 | 1010 | IL10RA | 1549 | OR4C4 6 | 2088 | SEMA6 D | 2627 | TSPAN 33 |
| 472 | CNTN AP2 | 1011 | IL10RB | 1550 | OR4D1 | 2089 | SEMA7 A | 2628 | TTYH1 |
| 473 | CNTN AP3 | 1012 | IL11RA | 1551 | OR4D2 | 2090 | SERIN C1 | 2629 | TTYH2 |
| 474 | CNTN AP3B | 1013 | IL12RB 1 | 1552 | OR4D5 | 2091 | SERIN C2 | 2630 | TTYH3 |
| 475 | CNTN AP4 | 1014 | IL12RB 2 | 1553 | OR4D6 | 2092 | SERIN C3 | 2631 | TXND C15 |
| 476 | CNTN AP5 | 1015 | IL13RA 1 | 1554 | OR4D9 | 2093 | SERIN C4 | 2632 | TYR |
| 477 | CORIN | 1016 | IL13RA 2 | 1555 | OR4D1 0 | 2094 | SERIN C5 | 2633 | TYRO3 |
| 478 | CPD | 1017 | IL15RA | 1556 | OR4D1 1 | 2095 | SEZ6 | 2634 | TYRP1 |
| 479 | CPM | 1018 | IL17RA | 1557 | OR4E1 | 2096 | SEZ6L 2 | 2635 | UBAC2 |
| 480 | CR1 | 1019 | IL17RB | 1558 | OR4E2 | 2097 | SGCA | 2636 | UGT8 |
| 481 | CR2 | 1020 | IL17RC | 1559 | OR4F3 | 2098 | SGCB | 2637 | ULBP1 |
| 482 | CRB1 | 1021 | IL17RD | 1560 | OR4F4 | 2099 | SGCD | 2638 | ULBP2 |
| 483 | CRB2 | 1022 | IL17RE | 1561 | OR4F5 | 2100 | SGCE | 2639 | ULBP3 |
| 484 | CRB3 | 1023 | IL18R1 | 1562 | OR4F6 | 2101 | SGCZ | 2640 | UMOD |
| 485 | CRHR 1 | 1024 | IL18RA P | 1563 | OR4F1 5 | 2102 | SHISA 4 | 2641 | UMOD L1 |
| 486 | CRHR 2 | 1025 | IL20RA | 1564 | OR4F1 6 | 2103 | SHISA 6 | 2642 | UNC5A |
| 487 | CRIM1 | 1026 | IL20RB | 1565 | OR4F1 7 | 2104 | SHISA 7 | 2643 | UNC5B |
| 488 | CRLF2 | 1027 | IL21R | 1566 | OR4F2 1 | 2105 | SHISA 8 | 2644 | UNC5 C |
| 489 | CRTA M | 1028 | IL22RA 1 | 1567 | OR4F2 9 | 2106 | SHISA 9 | 2645 | UNC5 D |
| 490 | CSF1 | 1029 | IL23R | 1568 | OR4K1 | 2107 | SHISA L1 | 2646 | UNC93 A |
| 491 | CSF1R | 1030 | IL27RA | 1569 | OR4K2 | 2108 | SIDT1 | 2647 | UNC93 B1 |
| 492 | CSF2R A | 1031 | IL31RA | 1570 | OR4K3 | 2109 | SIDT2 | 2648 | UPK1A |
| 493 | CSF2R B | 1032 | ILDR1 | 1571 | OR4K5 | 2110 | SIGIR R | 2649 | UPK1B |
| 494 | CSF3R | 1033 | IMPG2 | 1572 | OR4K13 | 2111 | SIGLE C1 | 2650 | UPK2 |
| 495 | CSMD 1 | 1034 | INSR | 1573 | OR4K1 4 | 2112 | SIGLE C5 | 2651 | UPK3A |
| 496 | CSMD 2 | 1035 | INSRR | 1574 | OR4K1 5 | 2113 | SIGLE C6 | 2652 | UPK3B |
| 497 | CSPG4 | 1036 | ISLR2 | 1575 | OR4K1 7 | 2114 | SIGLE C7 | 2653 | UPK3B L1 |
| 498 | CSPG5 | 1037 | ITFG1 | 1576 | OR4L1 | 2115 | SIGLE C8 | 2654 | USH2A |
| 499 | CTLA4 | 1038 | ITGA1 | 1577 | OR4M 1 | 2116 | SIGLE C9 | 2655 | UTS2R |
| 500 | CTNS | 1039 | ITGA2 | 1578 | OR4M 2 | 2117 | SIGLE C10 | 2656 | VASN |
| 501 | CUZD1 | 1040 | ITGA2 B | 1579 | OR4N2 | 2118 | SIGLE C11 | 2657 | VCAM 1 |
| 502 | CX3CL 1 | 1041 | ITGA3 | 1580 | OR4N4 | 2119 | SIGLE C12 | 2658 | VIPR1 |
| 503 | CX3CR 1 | 1042 | ITGA4 | 1581 | OR4N5 | 2120 | SIGLE C14 | 2659 | VIPR2 |
| 504 | CXAD R | 1043 | ITGA5 | 1582 | OR4P4 | 2121 | SIGLE C15 | 2660 | VLDLR |
| 505 | CXCL1 6 | 1044 | ITGA6 | 1583 | OR4Q2 | 2122 | SIGLE C16 | 2661 | VN1R1 |
| 506 | CXCR1 | 1045 | ITGA7 | 1584 | OR4Q3 | 2123 | SIGLE CL1 | 2662 | VN1R2 |
| 507 | CXCR2 | 1046 | ITGA8 | 1585 | OR4S1 | 2124 | SIRPA | 2663 | VN1R3 |
| 508 | CXCR3 | 1047 | ITGA9 | 1586 | OR4S2 | 2125 | SIRPB 1 | 2664 | VN1R4 |
| 509 | CXCR4 | 1048 | ITGA1 0 | 1587 | OR4X1 | 2126 | SIRPB 2 | 2665 | VNN1 |
| 510 | CXCR5 | 1049 | ITGA11 | 1588 | OR4X2 | 2127 | SIRPG | 2666 | VNN2 |
| 511 | CXCR6 | 1050 | ITGAD | 1589 | OR5A1 | 2128 | SIT1 | 2667 | VNN3 |
| 512 | CYBB | 1051 | ITGAE | 1590 | OR5A2 | 2129 | SLAMF 1 | 2668 | VSIG1 |
| 513 | CYSLT R1 | 1052 | ITGAL | 1591 | OR5A C1 | 2130 | SLAMF 6 | 2669 | VSIG2 |
| 514 | CYSLT R2 | 1053 | ITGAM | 1592 | OR5A C2 | 2131 | SLAMF 7 | 2670 | VSIG8 |
| 515 | DAG1 | 1054 | ITGAV | 1593 | OR5AK 2 | 2132 | SLAMF 8 | 2671 | VSIG1 0 |
| 516 | DAGLA | 1055 | ITGAX | 1594 | OR5AL 1 | 2133 | SLAMF 9 | 2672 | VSIG1 0L |
| 517 | DAGLB | 1056 | ITGB1 | 1595 | OR5A N1 | 2134 | SLC1A 1 | 2673 | VSIR |
| 518 | DCBLD 1 | 1057 | ITGB2 | 1596 | OR5AP 2 | 2135 | SLC1A 2 | 2674 | VSTM1 |
| 519 | DCBLD 2 | 1058 | ITGB3 | 1597 | OR5A R1 | 2136 | SLC1A 3 | 2675 | VSTM4 |
| 520 | DCC | 1059 | ITGB4 | 1598 | OR5AS 1 | 2137 | SLC1A 4 | 2676 | VSTM5 |
| 521 | DCHS1 | 1060 | ITGB5 | 1599 | OR5A U1 | 2138 | SLC1A 5 | 2677 | VTCN1 |
| 522 | DCHS2 | 1061 | ITGB6 | 1600 | OR5B2 | 2139 | SLC1A 6 | 2678 | XCR1 |
| 523 | DCSTA MP | 1062 | ITGB7 | 1601 | OR5B3 | 2140 | SLC1A 7 | 2679 | XKR3 |
| 524 | DCT | 1063 | ITGB8 | 1602 | OR5B1 2 | 2141 | SLC2A 1 | 2680 | XPNPE P2 |
| 525 | DDR1 | 1064 | ITLN1 | 1603 | OR5B1 7 | 2142 | SLC2A 2 | 2681 | ZACN |
| 526 | DDR2 | 1065 | ITM2B | 1604 | OR5B2 1 | 2143 | SLC2A 3 | 2682 | ZAN |
| 527 | DGCR 2 | 1066 | ITM2C | 1605 | OR5C1 | 2144 | SLC2A 4 | 2683 | ZDHH C5 |
| 528 | DIRC2 | 1067 | IZUMO 1 | 1606 | OR5D1 3 | 2145 | SLC2A 5 | 2684 | ZDHH C11 |
| 529 | DISP1 | 1068 | IZUMO 1R | 1607 | OR5D1 4 | 2146 | SLC2A 6 | 2685 | ZDHH C11B |
| 530 | DISP2 | 1069 | IZUMO 3 | 1608 | OR5D1 6 | 2147 | SLC2A 7 | 2686 | ZFYVE 27 |
| 531 | DISP3 | 1070 | JAG1 | 1609 | OR5D1 8 | 2148 | SLC2A 8 | 2687 | ZNRF4 |
| 532 | DLK1 | 1071 | JAG2 | 1610 | OR5F1 | 2149 | SLC2A 9 | 2688 | ZP1 |
| 533 | DLK2 | 1072 | JAM2 | 1611 | OR5G3 | 2150 | SLC2A 10 | 2689 | ZP2 |
| 534 | DLL1 | 1073 | JAM3 | 1612 | OR5H1 | 2151 | SLC2A 11 | 2690 | ZP3 |
| 535 | DLL3 | 1074 | JAML | 1613 | OR5H2 | 2152 | SLC2A 12 | 2691 | ZP4 |
| 536 | DLL4 | 1075 | KCNA3 | 1614 | OR5H6 | 2153 | SLC2A 13 | 2692 | ZPLD1 |
| 537 | DNER | 1076 | KCNG 4 | 1615 | OR5H1 4 | 2154 | SLC2A 14 | - | - |
| 538 | DPEP1 | 1077 | KCNJ3 | 1616 | OR5H1 5 | 2155 | SLC3A 1 | - | - |
| 539 | DPEP2 | 1078 | KCNJ4 | 1617 | OR5I1 | 2156 | SLC3A 2 | - | - |

Among the genes shown in Table 1, 2,653 genes for which gRNA design is easy were selected, and up to six gRNAs per gene and a control gRNA were designed for a gRNA library containing a total of 15,678 gRNAs. Then, such a synthesized gRNA library was cloned into a lentiviral vector to construct a lentiviral vector expressing the gRNA. The lentiviral vector was then subjected to next-generation sequencing (NGS) to determine whether the gRNAs were well expressed.

### Example 3. Construction of Cas9/gRNA library cells

The lentiviral vector to which the gRNA library of Example 2 was introduced was introduced into the breast cancer cells (MDA-MB-468-cas9) of Example 1. By adjusting a multiplicity of infection (MOI) level to 0.3, one lentiviral vector was introduced per the breast cancer cell. Then, through puromycin selection, the breast cancer cells into which the gRNAs were not inserted were removed, thereby constructing Cas9/guide RNA library cells. By separating the genomic DNA of the constructed Cas9/guide RNA library cells and performing NGS thereon, it was confirmed that the gRNA library was inserted into the cells.

### Experimental Example 1. Sorting of cells that have lost binding ability to antibody by using magnetic activated cell sorting (MACS)

The Cas9/gRNA library cells of Example 3 were treated with trypsine and re-suspended in 150 µl of an MACS buffer solution at a final concentration of 2 x 10⁶ cells. Afterwards, together with 5 µg of the antibody, the Cas9/gRNA library cells were incubated at room temperature for 2 hours, and the incubated Cas9/gRNA library cells and MACS protein G microbeads (130-071-101) were bound at 4 °C for 30 minutes. Then, an MACS buffer solution (100 µl) was added to the resulting Cas9/gRNA library cells, and cell sorting was performed thereon by using LD columns (130-042-901). Accordingly, cells not labeled with the antibody and cells labeled with the antibody were collected and subjected to cell counting.

### Experimental Example 2. Confirmation of loss of binding ability to antibody upon gene deletion

To confirm the inserted gRNAs in each group of the separated cells, gRNA regions from the genomic DNA of the cells were subjected to PCR and sequencing by NGS, thereby confirming distribution of 15,678 gRNAs. For each of a total of 15,678 gRNAs, ratios thereof in a control group and an experimental group were calculated, and gRNAs that were increased in the experimental group over the control were screened. Then, genes targeted by the screened gRNAs were identified, and tracked which genes have lost binding ability to the antibody when knocked out.

### 2.1 MACS performed with cetuximab as binding antibody for EGFR surface protein, confirming screening of EGFR-deficient cells

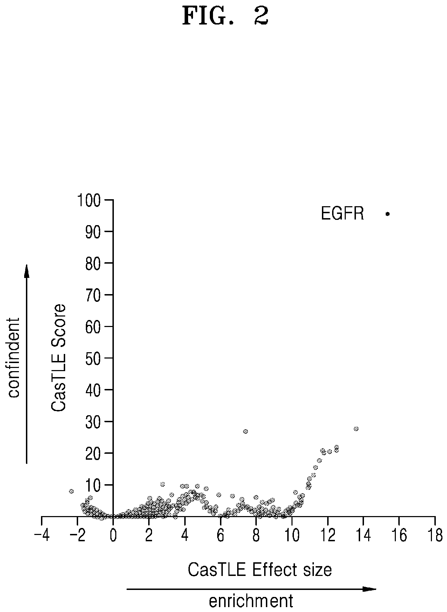

As a result of screening using cetuximab well known as a binding antibody for an epidermal growth factor receptor (EGFR) which is a cell surface protein, it was confirmed that gRNAs for EGFR-targeting guide sequences (e.g., SEQ ID NO: 1: TGTCACCACATAATTACCTG, SEQ ID NO: 2: GTGGAGCCTCTTACACCCAG, SEQ ID NO: 3: GTCTGCGTACTTCCAGACCA, SEQ ID NO: 4: TCTTGCCGGAATGTCAGCCG, SEQ ID NO: 5: CCTCATTGCCCTCAACACAG, and SEQ ID NO: 6: CTCTTCTTAGACCATCCAGG) were amplified more than 22,000-fold in cells not labeled with cetuximab, and these results are shown in FIG. 2.

FIG. 2 is a graph showing the results of performing MACS for the Cas9/guide RNA library cells by using cetuximab as an antibody, confirming the gRNAs highly expressed in cells not labeled with cetuximab over cells labeled with cetuximab.

As shown in FIG. 2, it was confirmed that gRNAs targeting the EGFR, which is an antigen for cetuximab, were highly expressed in cells not labeled with cetuximab.

As such, the gRNAs amplified in the cells not labeled with the antibody were confirmed and genes targeted by the amplified gRNAs were accordingly identified, indicating that the antigen to which the antibody binds can be identified.

### 2.2 MACS performed with CD44 antibody as binding antibody for CD44, confirming screening of CD44-deficient cells

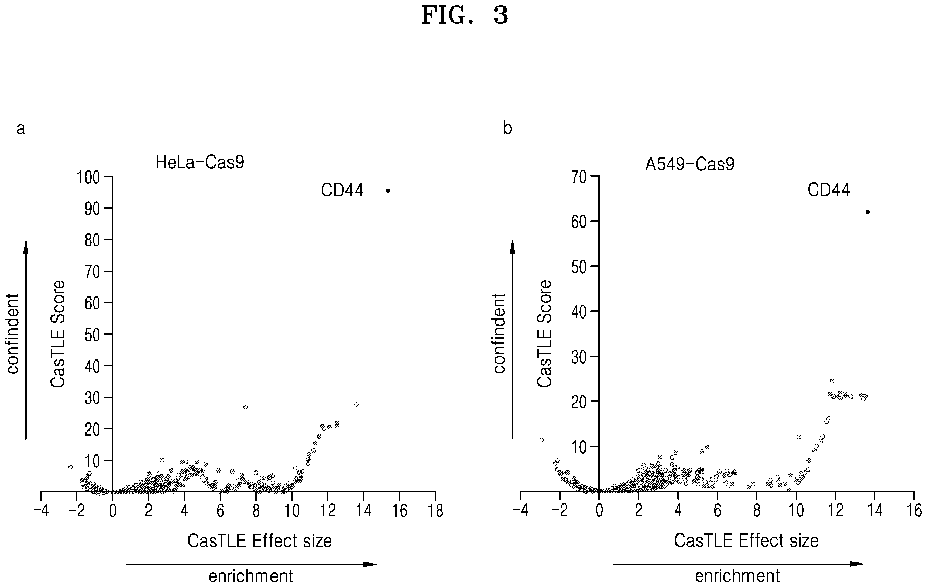

As a results of screening using a CD44 antibody as a binding antibody for a CD44 surface protein, it was confirmed that, in two different cell lines (HeLa and A549), gRNAs for CD44-targeting guide sequences (e.g., SEQ ID NO: 7: CATCACGGTTAACAATAGCT, SEQ ID NO: 8: AAGACTCCCATTCGACAACA, SEQ ID NO: 9: TGCTACTTCAGACAACCACA, SEQ ID NO: 10: TCGCTACAGCATCTCTCGGA, SEQ ID NO: 11: CGTGGAATACACCTGCAAAG, and SEQ ID NO: 12: CTACAGCATCTCTCGGACGG) were amplified in cells not labeled with the CD44 antibody, and these results are shown in FIG. 3.

FIG. 3 is a graph showing the results of performing MACS by using a CD44 antibody, confirming gRNAs, which are highly expressed in cells not labeled with the CD44 antibody, in different cell lines, wherein

FIG. 3A is a graph confirming gRNAs, which are highly expressed in cells not labeled with the CD44 antibody, in a HeLa cell line, and FIG. 3B is a graph confirming gRNAs, which are highly expressed in cells not labeled with the CD44 antibody, in an A549 cell line.

As shown in FIG. 3, it was confirmed that the CD44-targeting gRNAs were highly expressed in cells not labeled with the CD44 antibody.

As such, the gRNAs amplified in the cells not labeled with the antibody were confirmed and genes targeted by the amplified gRNAs were accordingly identified, indicating that the antigen to which the antibody binds can be identified.

### Experimental Example 3. Identification of antigens for anticancer antibodies

### 3.1 Discovery of novel antibodies derived from patients

Novel antibodies, S4-2 and S3-5, were discovered through a screening process on a patient-derived antibody library. Specifically, PBMCs were obtained from the blood of patients who were selected on the basis of clinical information and submitted consents, and RNAs were purified therefrom. Then, a cDNA library for producing antibodies was secured in the form of a single chain by using a PCR method, and then cloned into a phagemid. The antibody library thus secured was bound to cancer cells by using a phage display technique, so as to discover new antibodies that bind specifically to the cancer cells.

### 3.2 Identification of antigens for anticancer antibodies

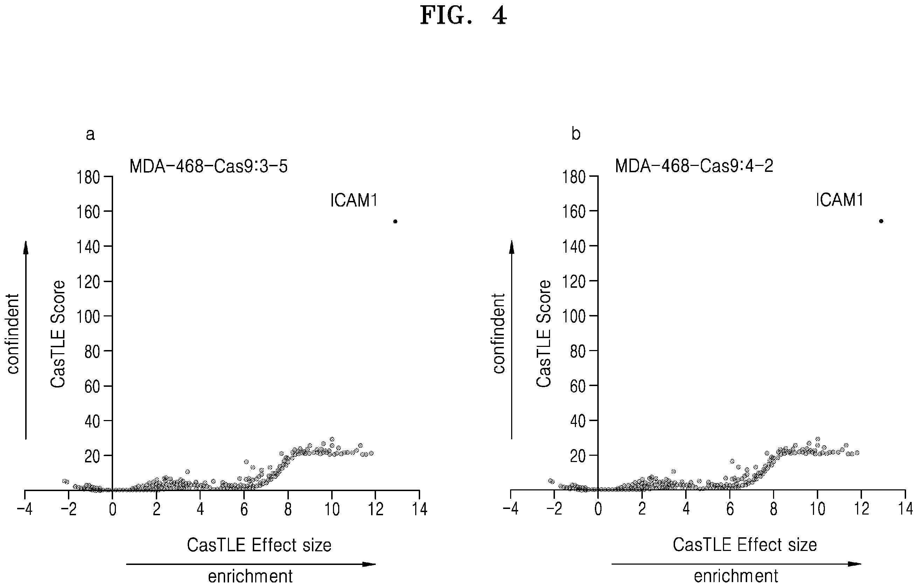

The same experiment as Experimental Example 2 was performed on the anticancer antibodies, S4-2 and S3-5, discovered from a patient-derived antibody library, and as a result, intercellular adhesion molecule 1 (ICAM-1) was identified as an antigen for the new anticancer antibodies. These results are shown in FIG. 4.

FIG. 4 is a graph showing the results of performing MACS for Cas9/guide RNA library cells by using, as antibodies, S4-2 and S3-5 anticancer antibodies discovered from a patient-derived antibody library, confirming gRNAs that are highly expressed in cells not labeled with the S4-2 or S3-5 anticancer antibody over cells labeled with the S4-2 or S3-5 anticancer antibody, wherein

FIG. 4A is a graph confirming guide RNAs highly expressed in cells not labeled with the S4-2 anticancer antibody discovered from a patient-derived antibody library, and FIG. 4B is a graph confirming guide RNAs highly expressed in cells not labeled with the S3-5 anticancer antibody discovered from a patient-derived antibody library.

As shown in FIG. 4, it was confirmed that gRNAs for ICAM1-targeting guide sequences (e.g., SEQ ID NO: 13: TGACGTGTGCAGTAATACTG, SEQ ID NO: 14: GCCCGCTGAGGTCACGACCA, SEQ ID NO: 15: CGGGCTGTTCCCAGTCTCGG, SEQ ID NO: 16: TGCAGGGACTCCAGAACGGG, SEQ ID NO: 17: ACCAGCACGGAGCCTCCCCG, and SEQ ID NO: 18: GCTCAGTTACTCACAGTACA) were highly expressed in cells not labeled with the S4-2 and S3-5 anticancer antibodies discovered from the patient-derived antibody library.

These results indicate that the ICAM1 is an antigen for the S4-2 and S3-5 anticancer antibodies.

### 3.3 Confirmation of binding of S4-2 and S3-5 anticancer antibodies to ICAM1-expressing cell line

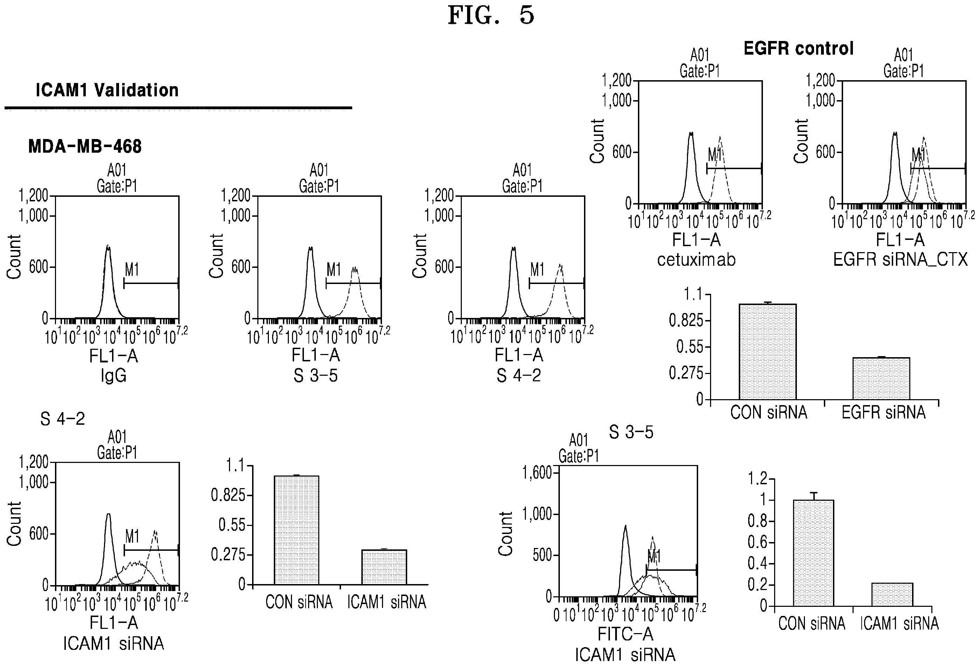

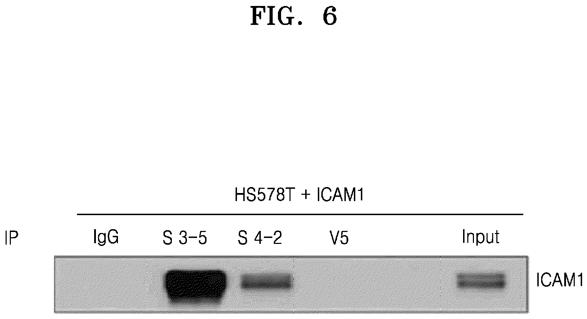

To confirm whether the ICAM1 directly binds to the S4-2 and S3-5 anticancer antibodies, ICAM1-specific siRNA was treated, and the loss of binding ability to the antibodies was confirmed by fluorescence-activated cell sorting (FACS). In addition, through immunoprecipitation-western blot analysis, it was tested whether the ICAM1 directly binds to the S4-2 and S3-5 antibodies. Then, the results are shown in FIGS. 5 and 6.

**[Table 2]**

| ICAM-1-specific siRNA | | Nucleotide sequence | SEQ ID NO: |
|---|---|---|---|
| ICAM1 | Sense | CCGGUAUGAGAUU GUCAUCAUUU | SEQ ID NO: 19 |
| | Antisense | AUGAUGACAAUCU CAUACCGGUU | SEQ ID NO: 20 |

FIG. 5 is a graph showing the results of performing fluorescence-activated cell sorting (FACS) after treating an MDA-MB-468 cell line with ICAM1-specific siRNAs.

FIG. 6 is an image obtained by performing immunoprecipitation-western blotting on an HS578T breast cancer cell line expressing ICAM1.

As shown in FIGS. 5 and 6, it was confirmed that the cell line not expressing the ICAM1 has lost the binding ability to the S4-2 and S3-5 anticancer antibodies, whereas the cell line expressing the ICAM1 binds to the S4-2 and S3-5 anticancer antibodies.

### 3.4 Confirmation of direct binding of ICAM 1 to S4-2 and S3-5 anticancer antibodies

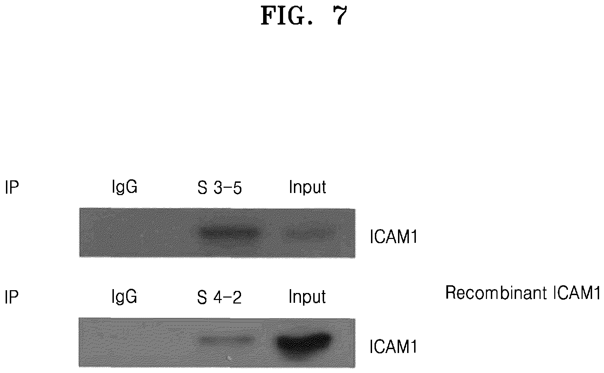

To confirm whether the ICAM1 directly binds to the S4-2 and S3-5 antibodies, purified ICAM1 was mixed with antibodies (IgG, 3-5, and 4-2) in vitro and subjected to immunoprecipitation-western blotting analysis. Then, the results are shown in FIG. 7.

FIG. 7 is an image obtained by performing immunoprecipitation-western blotting to determine whether ICAM1 directly binds to S4-2 and S3-5 anticancer antibodies.

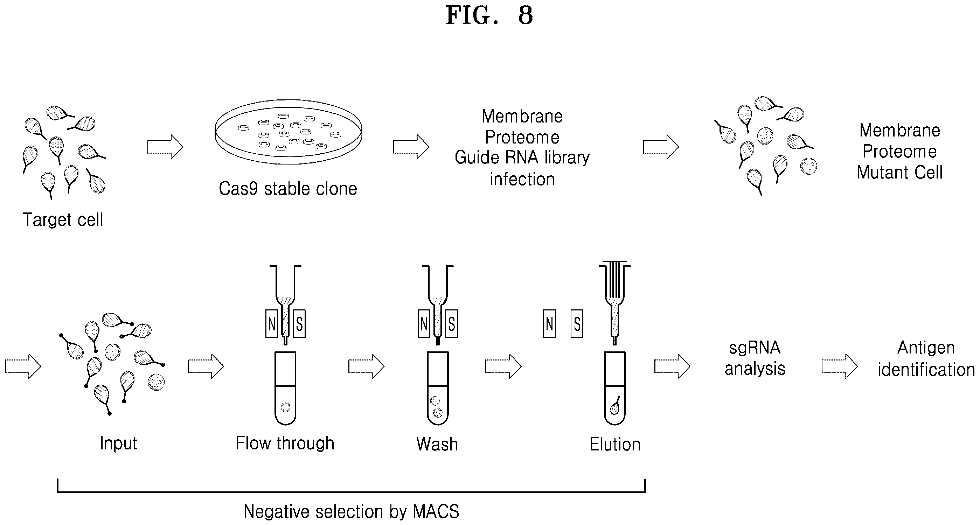

FIG. 8 is a schematic diagram explaining a method for screening cell surface antigens.

As shown in FIG. 7, it was confirmed that both S4-2 and S3-5 anticancer antibodies were directly bound to the ICAM1.

## Claims

1. A method for screening cell surface antigens, comprising:
treating separated cells with a vector to which a guide RNA (gRNA) library for cell surface proteins of the separated cells is introduced to produce vector-treated cells;
treating the vector-treated cells with a protein having binding ability to the separated cells to produce protein-treated cells; and obtaining, from the protein-treated cells, cells that have lost binding ability to the protein used in the treating.

2. The method of claim 1, wherein the protein having binding ability to the separated cells is a protein binding specifically to the cell surface proteins.

3. The method of claim 2, wherein the protein binding specifically to the cell surface proteins is any one selected from the group consisting of an antibody, an affibody, and a diabody.

4. The method of claim 3, wherein the antibody is discovered by screening a patient-derived antibody library.

5. The method of claim 1, wherein the separated cells are cancer cells.

6. The method of claim 1, wherein the separated cells include a Cas9 nuclease.

7. The method of claim 1, wherein the vector is a viral vector.

8. The method of claim 1, wherein, in the vector-treated cells, one vector is introduced per the separated cell.

9. The method of claim 1, wherein the gRNA library includes 1 to 10 gRNAs per gene of the cell surface proteins.

10. The method of claim 1, further comprising:
analyzing the gRNA contained in the cells that have lost binding ability to the protein that is used in the treating; and
identifying a gene targeted by the analyzed gRNA.

11. The method of claim 1, wherein the obtaining of the cells that have lost binding ability to the protein used in the treating comprises:
treating the protein-treated cells with a bead with a surface that binds to the protein that is used in the treating; and
collecting cells that do not bind to the bead.

12. The method of claim 10, further comprising:
preparing a control cell in which the gene targeted by the analyzed gRNA is knocked down or knocked out; and
treating the control cell with an antibody to measure whether an antigen-antibody reaction occurs.

13. The method of claim 12, wherein the antigen-antibody reaction is measured by using any one selected from the group consisting of enzyme-linked immunosorbent assay, radioimmunoassay, sandwich assay, western blotting, immunoprecipitation, immunohistochemical staining, fluorescent immunoassay, enzyme-substrate chromogenic assay, and antigen-antibody agglutination.

14. The method of claim 1, wherein the cell surface proteins include a tumor-associated antigen (TAA).
